# EUROPEAN PATENT APPLICATION

(11) **EP 2 144 021 A1**
(43) Date of publication of application: **13.01.2010**
(21) Application number: 08738609.0
(22) Date of filing: 17.04.2008
(51) Int. Cl.: F25D 23/00, A61L 9/01, A61L 9/16, F24F 1/00, F24F 7/00

(54) **REFRIGERATOR, AND DISINFECTING DEVICE**

(30) Priority: 20.04.2007 JP 2007111296; 27.04.2007 JP 2007118692; 27.04.2007 JP 2007118702; 30.07.2007 JP 2007197101; 30.07.2007 JP 2007197102; 30.07.2007 JP 2007197103; 30.07.2007 JP 2007197104; 30.07.2007 JP 2007197105; 30.07.2007 JP 2007197110; 30.07.2007 JP 2007197112
(71) Applicant: Panasonic Corporation, Kadoma-shi Osaka 571-8501 (JP)
(72) Inventor: YUASA, Masashi, Osaka 540-6207 (JP); TSUJIMOTO, Kahoru, Osaka 540-6207 (JP); KAWASAKI, Tatsuya, Osaka 540-6207 (JP); NISHIHATA, Hideo, Osaka 540-6207 (JP); OSHIMA, Atsuhiro, Osaka 540-6207 (JP); TAKASE, Kaiichi, Osaka 540-6207 (JP); MORIUCHI, Toshiyuki, Osaka 540-6207 (JP); IMADA, Hironori, Osaka 540-6207 (JP); FUJIHASHI, Makoto, Osaka 540-6207 (JP); HONDA, Kimiyasu, Osaka 540-6207 (JP); KIMURA, Yoshito, Osaka 540-6207 (JP); TATSUMU, Yoshikimi, Osaka 540-6207 (JP)
(74) Representative: Grünecker, Kinkeldey, Stockmair & Schwanhäusser Anwaltssozietät
(86) International application number: PCT/JP2008/001007
(87) International publication number: WO 2008/132815

(57) **Abstract**

In order to efficiently deodorize and sterilize an inside of a refrigerator, the refrigerator includes a heat-insulating main body which is made of a heat-insulating material and in which a storage compartment is formed, a door attached to an opening provided on the heat-insulating main body so as to be openable and closable, and a cooling unit which cools air inside the heat-insulating main body, and the refrigerator includes: a support which is placed in the storage compartment and on which a photocatalyst is supported; an irradiation unit which irradiates the support with excitation light which excites the photocatalyst; and an air blowing unit which forcibly blows the cool air inside the storage compartment toward the support.

## Description

### Technical Field

The present invention relates to a refrigerator which cools the air inside a storage compartment directly and indirectly, and particularly relates to a refrigerator and a sterilization apparatus which can sterilize and deodorize an inside of the storage compartment.

### Background Art

Conventionally, for a compact refrigerator and a refrigerator used in Europe and other arid areas, it is not considered very important to take measures against dew condensation generated in the storage compartment, and thus a commonly used method is a direct cooling method in which a direct cooling panel or the like is used for cooling the air in the storage compartment. For example, Patent Reference 1 discloses a refrigerator for which the direct cooling method is used.

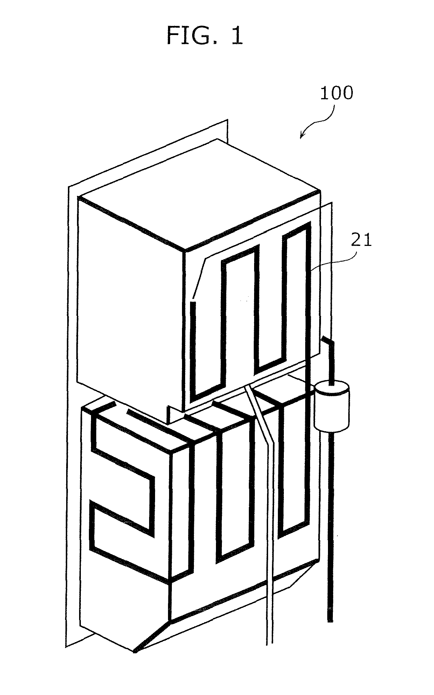

FIG. 1 is a diagram showing a schematic view as seen from the back of a refrigerator with a heat-insulating portion removed.

As the figure shows, a refrigerator 100 includes a cooling pipe 21 having a serpentine shape inside the back wall of the storage compartment. The air inside the storage compartment is directly cooled with a liquid cooling agent circulating at low temperature within the cooling pipe 21.

In addition, Patent Reference 2 discloses a refrigerator, which can suppress propagation of bacteria and molds in the storage compartment.

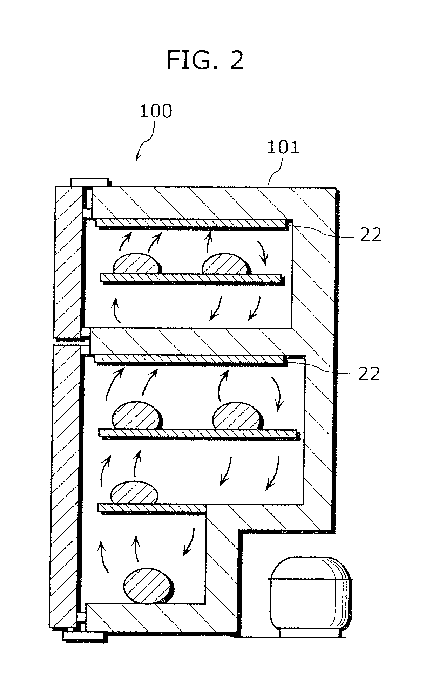

FIG. 2 is a cross-sectional diagram of the refrigerator having a function to suppress propagation of the bacteria and molds.

As the figure shows, in the refrigerator 100, a sheet 22 containing an organic volatile antibacterial agent is attached to the ceiling of the storage compartment. Then, it is intended to suppress the propagation of the bacteria and molds in the storage compartment through gradual evaporation of the organic volatile antibacterial agent from the sheet 22.

On the other hand, in recent years, since various foods from various areas are stored in the refrigerator, the need for removing odors generated from such foods stored in the refrigerator and the need for sterilizing the inside of the refrigerator are very high. Thus, a sterilization and deodorization apparatus has been actively developed for the purpose of deodorizing and sterilizing the inside of the refrigerator. A conventional sterilization and deodorization apparatus deodorizes and sterilizes the air passing though a filter provided in an air path. In addition, a conventional technique for photocatalytic deodorization and sterilization is to irradiate a filter containing titanium oxide with an ultraviolet ray so as to cause a photocatalytic reaction by which to perform deodorization and sterilization.

Hereinafter, the conventional sterilization and deodorization apparatus disclosed by Patent Reference 3 will be described with reference to the drawings.

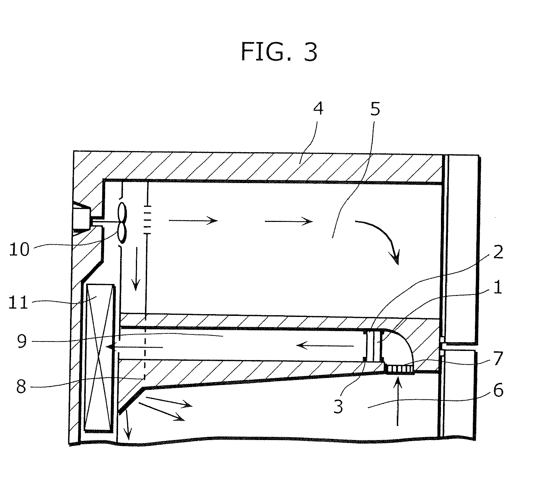

FIG. 3 is a partial longitudinal sectional view of the refrigerator when a sterilization and deodorization apparatus is attached to an air inlet portion for the air returning to the refrigerating compartment.

The sterilization and deodorization apparatus includes: a sterilization filter 1, a deodorization filter 2, and a fitting frame 3. Here, the sterilization filter 1 has a honeycomb shape, made of silver-compounded zeolite composed of oxides such as silicon, aluminum, and sodium. The sterilization filter 1 has approximately: 100 to 250 cells/square inch, an aperture ratio of 70 to 80%, and a thickness of 8 mm.

The deodorization filter 2 is a blend of manganese oxide and an oxide of silicon or aluminum, which is kneaded into a honeycomb shape, and has, in this case, almost the same number of cells and aperture rate as the deodorization filter described above. These sterilization filter 1 and deodorization filter 2 are fixed together with the fitting frame 3.

The figure shows part of a cross-sectional structure of the refrigerator, focusing on an inlet portion 7 through which cool return air is sucked, to which the sterilization and deodorization apparatus is mounted. In the figure, a freezing compartment 5 is disposed in an upper part and a refrigerating compartment 6 is disposed in a lower part, and a cooler 11 is disposed in the back surface of the freezing compartment 5 and the refrigerating compartment 6. In addition, a cool-air path 9 is provided in an insulating portion 8 provided between the freezing compartment 5 and the refrigerating compartment 6. In the cool-air path 9, the sterilization filter 1 is provided on the side of the inlet portion 7 and the deodorization filter 2 is provided at the back of the sterilization filter 1.

The operation of the refrigerator thus configured will be described below.

The cool air generated by the cooler 11 partially flows into the freezing compartment 5, and partially flows into the refrigerating compartment 6 and others below. The cool air after circulating in each unit passes through the inlet portion 7 through which the return air is sucked, and flows toward the cooler 11 via the cool-air path 9. At this time, the wind speed in the cool-air path 9 is approximately 0.5 m/sec. By providing the sterilization and deodorization apparatus, the sterilization filter 1 first captures bacteria and mold spores together with dust and dirt, and then the deodorization filter 2 promotes chemical change of odor components.

With such a structure in which a combination of the deodorization and sterilization filters is provided in the cool-air path, it is possible to realize a compact and clean refrigerator which reduces bacteria and bad odors through efficient performance of deodorization and sterilization.

In addition, for example, a refrigerator which is a combination of a deodorization filter 17 and an ion generator 16 is disclosed (see Patent Reference 4).

FIG. 4 is a center longitudinal sectional view of a conventional refrigerator disclosed in Patent Reference 4.

FIG. 5 is a center longitudinal sectional view of a sterilization and deodorization apparatus provided in the conventional refrigerator.

FIG. 6 is a top view of the sterilization and deodorization apparatus mounted in the conventional refrigerator.

This conventional refrigerator includes two storage compartments formed with a heat-insulating main body 101 and a heat-insulating door 107, and includes a refrigerating compartment 102 in an upper part and a freezing compartment 103 in a lower part. The refrigerating compartment 102 includes shelf plates 46 on which food is placed, and a cooler 110 dedicated for the refrigerating compartment is provided in a back wall at the back of the shelves. Likewise, in the freezing compartment 103, cases 48 for storing food are provided, and another cooler 110 dedicated for the freezing compartment is provided in an upper part of each of the cases 48. In addition, in a rear lower part of the heat-insulating main body 101, a compressor 114 of a refrigeration cycle is provided. In addition, a sterilization apparatus 200 is provided in the ceiling of the refrigerating compartment 102.

This sterilization apparatus 200 includes: a housing 250 having an inlet 251 which opens on a front surface and an outlet 252 which opens on a rear undersurface; an air blowing unit 203 which sucks air from the refrigerating compartment 102 via the inlet 251 and discharges the air through the outlet 252; an ion generator 16 which is provided in the outlet side of the air blowing unit 203 and generates sterilization ions; a deodorization filter 17 which is provided on the inlet side of the air blowing unit 203 and absorbs odor components in the air; and an internal light 18.

The operation of the thus-configured refrigerator will be described below.

First, the operation of cooling the refrigerating compartment 102 is described. The compressor 114, when in operation, cools the cooler 110 to a low temperature, so that the air in an upper back of the refrigerating compartment 102 is cooled.
Fresh air thus generated has high specific gravity, and so goes down along the back wall of the refrigerating compartment 102 to be distributed onto each of the shelf plates 46 to chill the food placed thereon. The cool air after cooling the food has low specific gravity, and so goes up along the inner wall of the insulating door 107 and returns to an uppermost part of the refrigerating compartment 102. The cool air having thus returned is cooled again by the cooler 110, and convectively flows inside the refrigerating compartment 102 as fresh air again. As described above, the method of cooling the storage compartment by using natural convective air is referred to as the direct cooling method.

Note that the operation of cooling the freezing compartment 103 is the same as in the case of the refrigerating compartment 102, and thus the detailed explanation thereof is omitted.

Next, the operation of deodorization and sterilization is described. Inside the refrigerating compartment 102 where natural convective air is generated, when the air blowing unit 203 equipped inside the sterilization apparatus 200 is operated, part of the cool air having retuned to the uppermost part of the refrigerating compartment 102 is absorbed through the inlet 251. The absorbed cool air first passes the deodorization filter 17, in which odor components are absorbed. Subsequently, the cool air having passed through the air blowing unit 203 is discharged through the outlet 252 along with ions having a sterilizing effect, which is generated by the operation of the ion generator 16. It is designed that the cool air containing such sterilization ions spreads to every corner of the refrigerating compartment 102 along with the natural convective air, and surrounds to kill the molds floating in the air.

On the other hand, the sterilization ions are liable to vanish when hitting a blade of the air blowing unit 203 and so on or to be absorbed by the deodorization filter 17 to decrease. Thus, in this sterilization apparatus 200, the decrease of the sterilization ions are prevented by placing the ion generator 16 downstream from the air blowing unit 203 and the deodorization filter 17.

In addition, the back wall surface of the refrigerating compartment 102, in which the cooler 110 is provided, is chronically wet with dew condensation water and thus has a risk of causing breakdown or electric leakage when the dew condensation water attaches to an electric component. Therefore, the sterilization apparatus 200 is kept from breakdown and electric leakage by disposing the ion generator 16 having a high-voltage electric circuit at a considerable distance from the back wall surface of the refrigerating compartment 102, that is, in a frontal part of the housing 250.

With the operation as described above, the cool air in the refrigerating compartment 102 is deodorized and sterilized, thereby keeping a clean state with reduced bad odor and bacteria.
Patent Reference 1: Japanese Unexamined Patent Application Publication No.7-120108
Patent Reference 2: Japanese Unexamined Patent Application Publication No. 10-262629
Patent Reference 3: Japanese Unexamined Patent Application Publication No. 5-157444
Patent Reference 4: Japanese Unexamined Patent Application Publication No. 2005-134075

### Disclosure of Invention

### Problems that Invention is to Solve

Meanwhile, in the case of a direct cooling refrigerator, there is a problem, for example, that the air cooled on the back surface of the storage compartment stays in a lower part of the storage compartment because of its high density, causing vertical temperature irregularity in the storage compartment. Specifically, such temperature irregularity is prominent in the storage compartment when shelf plates are provided for dividing the storage compartment into upper and lower parts, where food and so on are packed.

In addition, there is also a problem that the air stagnant in the storage compartment causes bacteria and molds to locally propagate in high density.

Furthermore, use of an organic volatile antibacterial agent has been suggested as a measure against the bacteria and molds, but there is a limit to the amount of evaporation of the antibacterial agent. Therefore, this necessitates a periodical replacement of the antibacterial agent, thus requiring extra care. In addition, for the refrigerator which cannot have convection of air inside the storage compartment, there is a possibility that the antibacterial agent stays only in part of the storage compartment and does not produce sufficient sterilization and other effects in the overall storage compartment. In addition, using an organic antibacterial agent in the storage compartment for storing food causes a problem in itself.

Moreover, a cooling pipe is placed in the back surface of the storage area, and a sheet containing an organic volatile antibacterial agent is placed almost covering the ceiling of the storage compartment. This causes another problem of difficulty in selecting where to provide a lighting unit which illuminates the inside of the storage compartment.

The present invention is conceived to solve the above problems, and it is an object of the present invention to provide a refrigerator which increases deodorization and antibacterial effects in the refrigerator and can diffuse these effects extensively in the storage compartment.

In addition, the present invention also has an object to provide a refrigerator which can generate a temperature appropriate for the photocatalyst used for sterilization and deodorization.

In addition, the present invention also has an object to provide a refrigerator which extensively produces deodorization and sterilization effects and can illuminate the inside of the storage compartment without forcible circulation of the cool air in the refrigerator.

In addition, the present invention also has an object to improve sterilization and deodorization efficiencies, to thereby prevent decrease in the capacity of the storage compartment.

Furthermore, after concentrated studies and experiments for increasing the sterilization and deodorization efficiencies, the inventors of the present invention have focused on the temperature gradient in the refrigerator, to arrive at an appropriate placement of the photocatalyst and so on.

Based on these, another object of the present invention is to provide a refrigerator which allows optimal placement which allows efficient performance of the deodorization and antibacterial effects.

On the other hand, in an indirect cooling refrigerator as described above, deodorization and sterilization filters are provided in the cool-air return path. This causes a problem that the cool air, once clean after passing thorough the deodorization and sterilization filters, comes to contain again various odors and bacteria around the machinery compartment while circulating in the cool-air path and the cooler, and is not clean any longer when it blows into the refrigerator.

In order to solve such conventional problems as described above, it is an object of the present invention to provide a clean refrigerator which has odorless cool air blown in and suppresses propagation of bacteria, by providing deodorization and sterilization filters in an outlet portion in a cool-air path.

In addition, in the indirect cooling refrigerator as described above, the deodorization and sterilization filters are provided blocking a return path of the cool air, thus becoming a large obstacle to the air path in the circulation path for the cool air. Thus, in order to obtain the same cooling performance as in the case of not using the filter, it is necessary to enhance the capacity of the air blower for forcibly circulating the cool air.

However, such enhancement of the capacity of the air blower is not preferable since it is incompatible with such problems as noise and energy saving.

The present invention has been conceived to solve such problems regarding the indirect cooling refrigerator described above, and it is an object of the present invention to provide a refrigerator which produces deodorization and antibacterial effects extensively while, at the same time, reducing as much pressure loss as possible in the circulation path of the cool air.

Furthermore, in the conventional configuration in which the above-described ion generator is used for sterilization and deodorization, the ion generator is provided in a frontal part of the housing in order to prevent troubles caused by dew condensation water attaching to the back wall surface of the refrigerating compartment. Thus, an air path from the ion generator to the outlet is provided longer than necessary, which makes it difficult to reduce the size of the sterilization and deodorization apparatus itself, causing a problem of poor usability in terms of food storage.

In addition, in the conventional structure described above, the ion generator requires a high-voltage electric circuit, causing a problem that it is necessary to fully consider a measure against refrigerant leakage in the case of mounting this apparatus to a refrigerator using a flammable refrigerant such as hydrogenated carbon (HC).

In addition, in the conventional configuration described above, the air blowing unit is provided inside the housing. This does not allow visual checking of the operation of the fan of the air blowing unit, causing a problem that it is not possible to timely check the trouble with the sterilization and deodorization apparatus.

The present invention has been conceived in view of this problem, and it is an object of the present invention to provide a user-friendly and highly-safe refrigerator by providing a sterilization and deodorization apparatus which is drip-proofed and compact, which does not need a high-voltage electric circuit, and which allows visual checking of the operating condition of the air blowing unit.

### Means to Solve the Problems

In order to achieve the above object, a refrigerator according to the present invention includes a heat-insulating main body which is made of a heat-insulating material and in which a storage compartment is formed, a door attached to an opening provided on the heat-insulating main body so as to be openable and closable, and a cooling unit which cools air inside the heat-insulating main body, and the refrigerator includes: a support which is placed in the storage compartment and on which a photocatalyst is supported; an irradiation unit which irradiates the support with excitation light which excites the photocatalyst; and an air blowing unit which forcibly blows the air inside the storage compartment toward the support.

With this, sterilization and deodorization is performed using the photocatalyst in the air flow which is generated by the air blowing unit, thereby making it possible to generate a convection current of sterilized and deodorized clean air in the storage compartment. Accordingly, it is possible to keep the whole storage compartment clean, and to preserve the food longer. Furthermore, since the air blown by the air blowing unit generates a forcible convection in the storage compartment, it is possible to remove temperature irregularity inside the storage compartment.

Thus, the refrigerator according to the present invention forcibly blows the air inside the storage compartment, sterilizes bacteria and so on contained in the air, and blows the sterilized air into the storage compartment. Accordingly, it is possible to sterilize the whole storage compartment by forcing convection of the air inside the storage compartment.

With this, two components, that is, the air blowing unit and the irradiation unit are the electric parts affected by dew condensation water, and since these two can be splash-proofed with a relatively easy measure, it is possible to collectively place the structural components of the sterilization apparatus including the air blowing unit and the irradiation unit in proximity to the back wall surface of the refrigerator, thereby providing a more compact deodorization and sterilization apparatus than in the conventional structure.

Furthermore, for both the air blowing unit and the irradiation unit, those operated with a direct-current and low-voltage electric circuit are widely used, and thus the refrigerator does not need any high-voltage electric circuit when these components are used.

Furthermore, it is preferable that a cover attached to an inner wall of the heat-insulating main body and covering the sterilization apparatus should also be included.

With this, it is possible to isolate the temperature inside the sterilization apparatus from the temperature inside the storage compartment, thereby making it possible to maintain the sterilization apparatus at a temperature more appropriate for photocatalysis. Accordingly, for example, even when humid air is introduced into the storage compartment when opening or closing the door of the refrigerator, it is possible to prevent generation of dew condensation on the support 201.

The refrigerator according to the present invention thermally isolates the sterilization apparatus from the inside of the storage compartment. Accordingly, it becomes possible to maintain the interior temperature of the sterilization apparatus at a temperature appropriate for photocatalysis, and also to prevent generation of dew condensation in the sterilization apparatus.

Furthermore, it is preferable that a lighting unit which is covered with the cover and illuminates an inside of the heat-insulating main body also be included.

With this, the sterilization apparatus and the lighting unit are covered with the same cover, and thus allowing the sterilization, deodorization, and lighting of the storage compartment in a compact state. In addition, it is possible to efficiently use the heat generated by the lighting unit for the sterilization and deodorization. In addition, this also allows sharing an electrical system with the lighting unit 300, thereby achieving space saving and cost reduction.

As described above, the refrigerator according to the present invention forcibly blows out the air inside the storage compartment, sterilizes bacteria and so on contained in the air, and blows the sterilized air into the storage compartment. Accordingly, it is possible to sterilize the whole storage compartment by forcing convection of the air inside the storage compartment. Furthermore, since the inside of the storage compartment is illuminated with the illumination unit, which is covered with the cover, along with the sterilization apparatus, it is possible to provide a compact sterilization and lighting unit.

The flow path forming unit has, in the flow path for the air, a narrow portion of which a cross-sectional area is narrower than in other portions, and the support is placed in the narrow portion, and the support, the irradiation unit, and the air blowing unit are attached in the flow path forming unit.

With this, the air, which is forced through a flow path by the air blowing unit 203, is narrowed in a narrow portion. Accordingly, the air efficiently contacts the support 201 provided in the narrow portion, thereby increasing the sterilization and deodorization effects.

Since the present invention allows sterilization of the inside of the storage compartment as a whole, it is possible to provide a refrigerator of higher quality.

In addition, the present invention allows provision of a sterilization apparatus which can increase the sterilization and deodorization effects.

The air blowing unit, the support, and the cooling unit are placed in order, starting from upstream of a flow of the air generated by the air blowing unit.

With this, the air having a relatively high temperature is allowed to contact the support 201, thereby preventing generation of dew condensation on the support 201. Accordingly, it is possible to suppress the deterioration of the photocatalytic function, thereby allowing efficient performance of sterilization and deodorization.

In addition, this placement, when adopted, does not disturb the natural convection generated by the cooling unit and thus allows formation of an air flow in a direction that promotes the natural convection, thereby making it possible to remove temperature irregularity within the storage compartment with high efficiency.

As described above, in the refrigerator according to the present invention, the air having a relatively high temperature is sent toward a lower-temperature side. With this, it is possible to reinforce the natural convection inside the storage compartment. In addition, the photocatalyst is reacted to the air having a relatively high temperature, thus allowing efficient performance of sterilization and deodorization.

In order to solve the above conventional problems, a refrigerator according to the present invention includes a heat-insulating main body in which a storage compartment is formed, a flow path forming unit which forcibly circulates air, a support on which a photocatalyst is supported, and an irradiation unit which irradiates the photocatalyst, and the support is placed in an outlet portion of the flow path forming unit.

With this, since the circulating air is deodorized and sterilized by the absorption filter immediately before blown into the refrigerator, it is possible to blow clean air into the refrigerator.

Furthermore, it is preferable to provide a switch for starting and stopping operation of the air blowing unit and the irradiation unit.

With this, when the switch is turned ON to the operation side, odors from food and bacteria inside the refrigerator are collected into the flow path forming unit to be deodorized and sterilized by the deodorization and sterilization filters before the cool air is blown into the storage compartment, thereby making it possible to blow clean air into the refrigerator.

In addition, it is preferable to provide the air blowing unit adjacent to the inlet.

According to this, it is possible to visually check the light of the irradiation unit from the gaps between blades. Accordingly, the user can directly see and check the rotation of the blades in the air blowing unit or the irradiation by the irradiation unit.

In addition, a sterilization apparatus according to the present invention is attached to a refrigerator including a heat-insulating main body which is made of a heat-insulating material and in which a storage compartment is formed, a door attached to an opening provided on the heat-insulating main body so as to be openable and closable, and a cooling unit which cools air inside the heat-insulating main body, and the sterilization apparatus includes: a support on which a photocatalyst is supported; an irradiation unit which irradiates the support with excitation light which excites the photocatalyst; an air blowing unit which forcibly blows the air inside the storage compartment toward the support, and a flow path forming unit which forms a flow path for the air blown by the air blowing unit, and the support, the irradiation unit, and the air blowing unit are attached to the flow path forming unit.

In addition to the characteristics described above, this makes it easier to attach or detach the sterilization apparatus to or from the refrigerator.

### Effects of the Invention

Since the present invention allows sterilization of an inside of a storage compartment as a whole, it is possible to provide a refrigerator of higher quality.

In addition, a sterilization apparatus according to the present invention allows easy attachment and detachment of the sterilization apparatus to the refrigerator, thus allowing provision of the sterilization apparatus which can sterilize the inside of the storage compartment as a whole.

In addition, since the refrigerator according to the present invention allows provision of a compact sterilization unit and an illumination unit, it is possible to use the storage compartment spaciously, thereby making it possible to provide a more usable refrigerator.

Since the refrigerator according to the present invention can perform sterilization and deodorization efficiently, it is possible to provide a higher-quality refrigerator which produces increased sterilization and deodorization effects.

In addition, the refrigerator according to the present invention increases a frequency of contact between the cool air and the deodorization and sterilization filters, thereby making it possible to provide a more usable, higher-quality refrigerator.

### Brief Description of Drawings

[FIG. 1] FIG. 1 is a diagram showing a schematic view as seen from the back of a refrigerator with a heat-insulating portion removed.
[FIG. 2] FIG. 2 is a cross-sectional diagram of the refrigerator having a function to suppress propagation of the bacteria and molds.
[FIG. 3] FIG. 3 is a partial longitudinal sectional view of the refrigerator when a sterilization and deodorization apparatus is provided in an air inlet portion for the air returning to the refrigerating compartment.

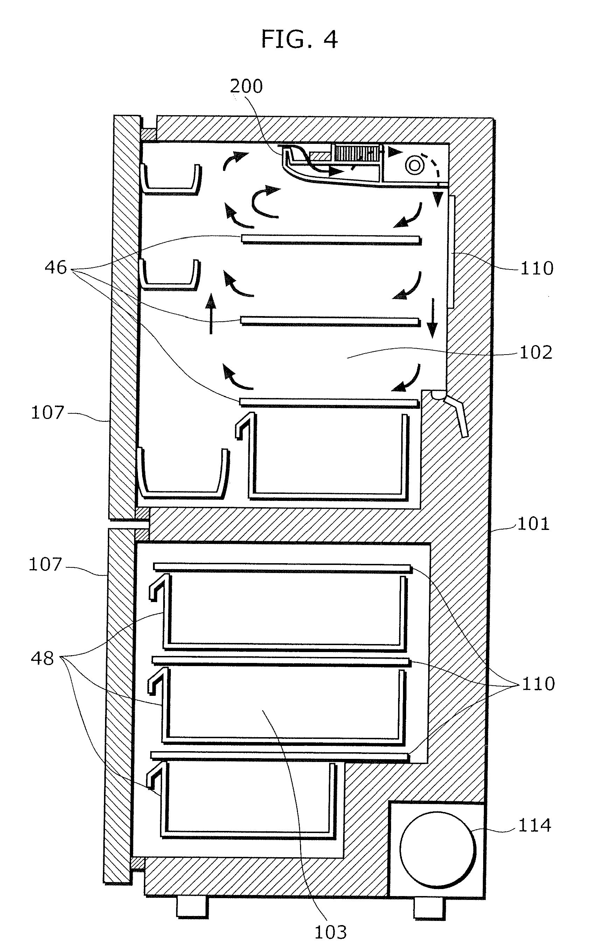

[FIG. 4] FIG. 4 is a center longitudinal sectional view of a conventional refrigerator disclosed in Patent Reference 4.

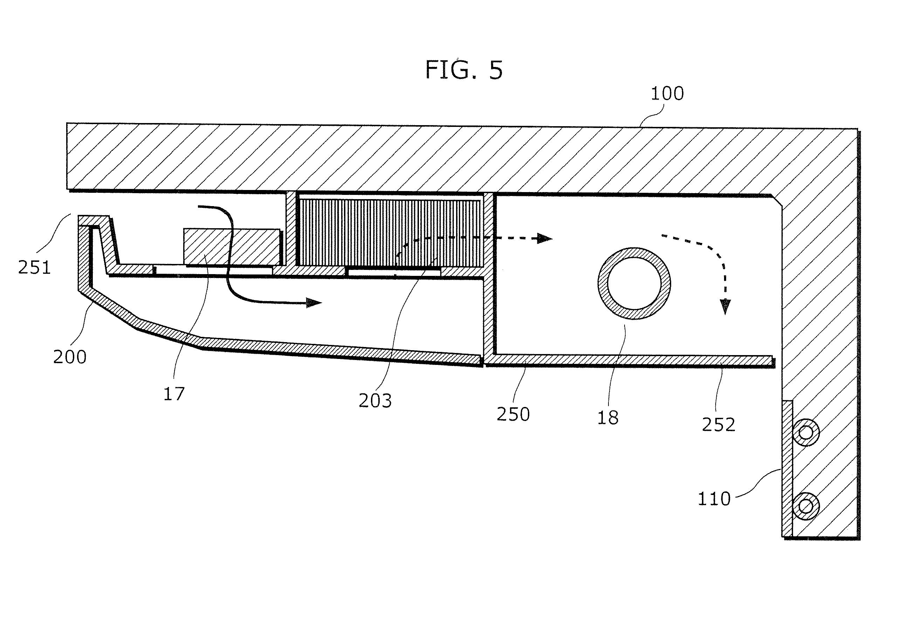

[FIG. 5] FIG. 5 is a center longitudinal sectional view of a sterilization and deodorization apparatus provided in the conventional refrigerator.

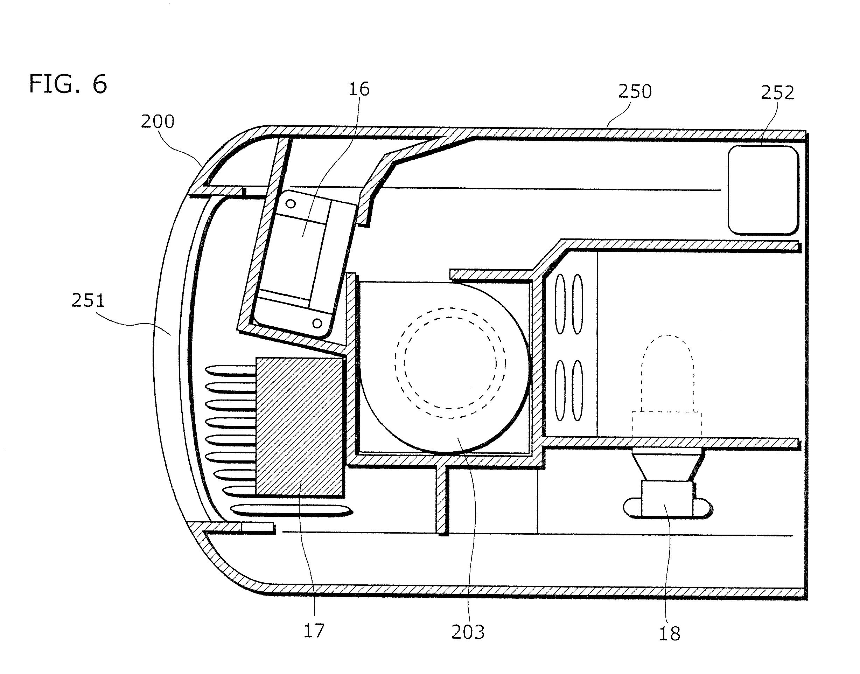

[FIG. 6] FIG. 6 is a top view of the sterilization and deodorization apparatus provided in the conventional refrigerator.

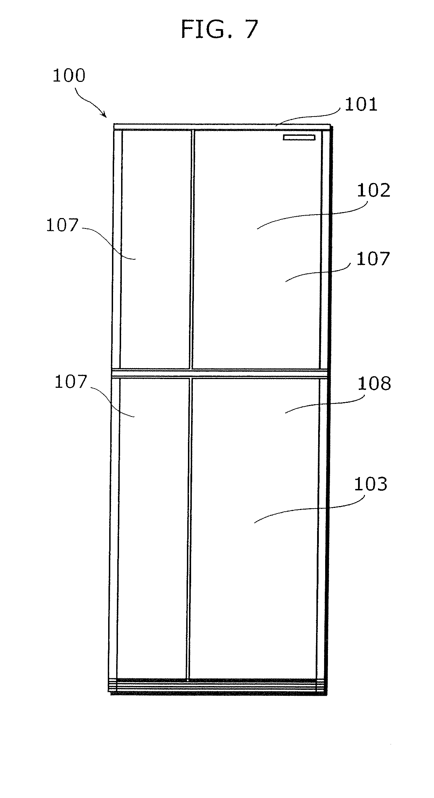

[FIG. 7] FIG. 7 is an elevation view of the refrigerator in a first embodiment of the present invention.

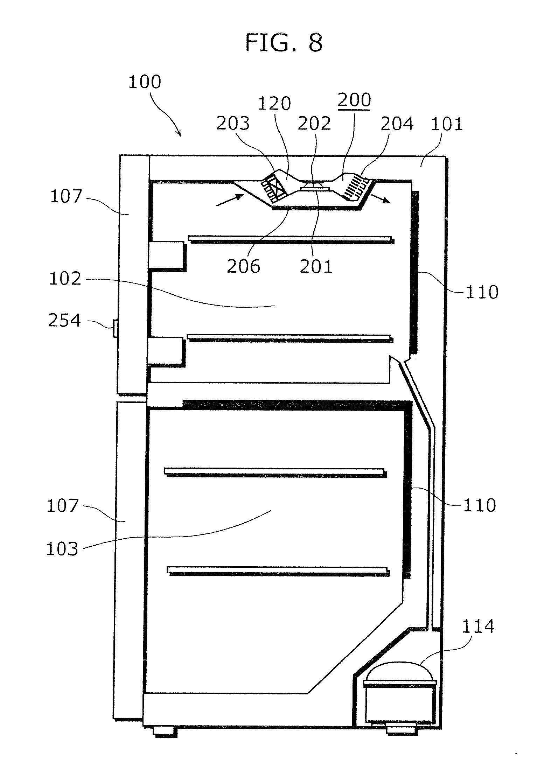

[FIG. 8] FIG. 8 is a longitudinal sectional view schematically showing the refrigerator in the present embodiment.

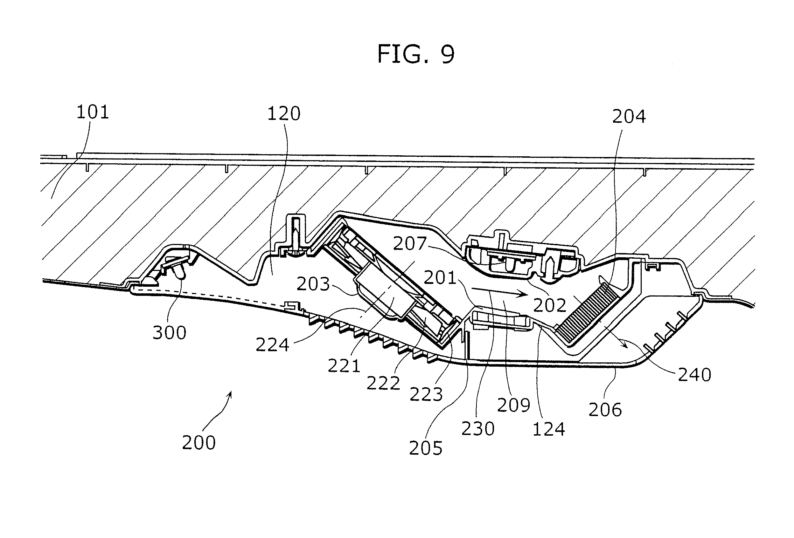

[FIG. 9] FIG. 9 is a cross sectional view of a sterilization apparatus mounted to the refrigerator.

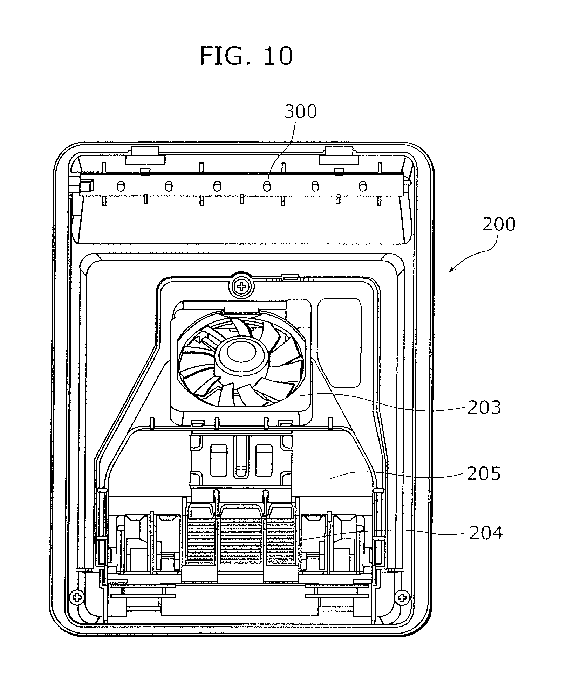

[FIG. 10] FIG. 10 is a bottom view of a sterilization apparatus mounted to the refrigerator.

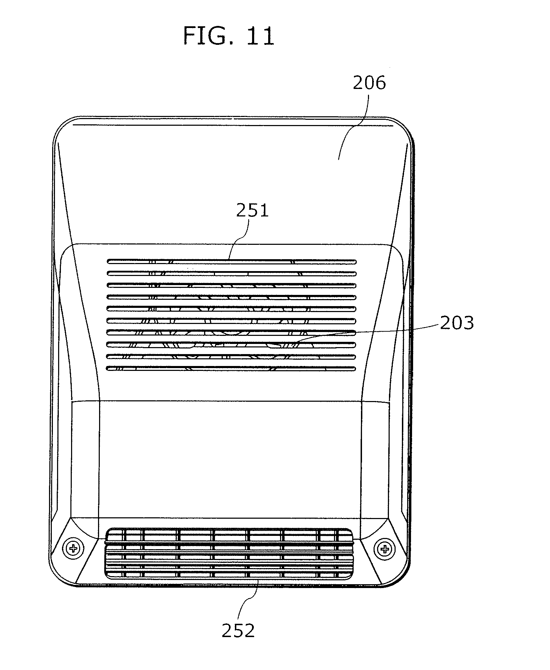

[FIG. 11] FIG. 11 is a bottom view showing the sterilization apparatus and a lighting unit, which are covered with a cover.

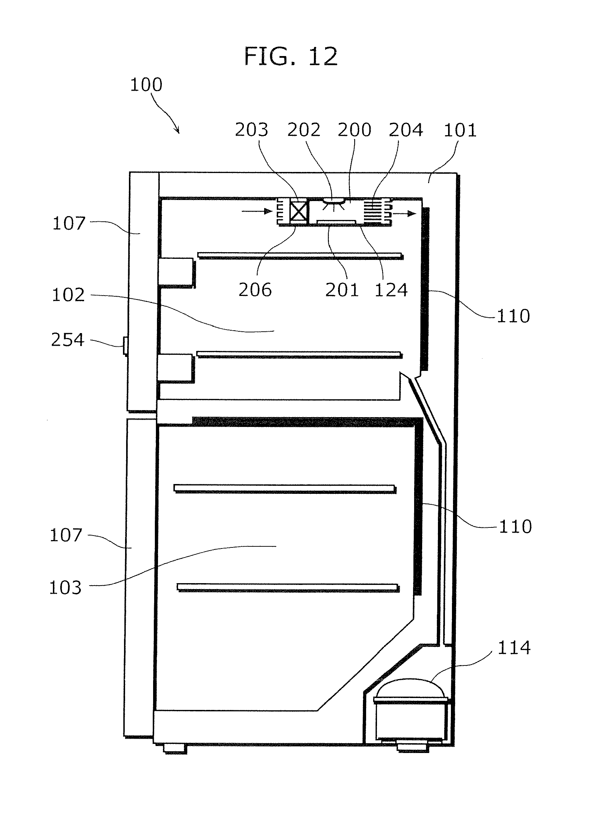

[FIG. 12] FIG. 12 is a longitudinal sectional view of a refrigerator.

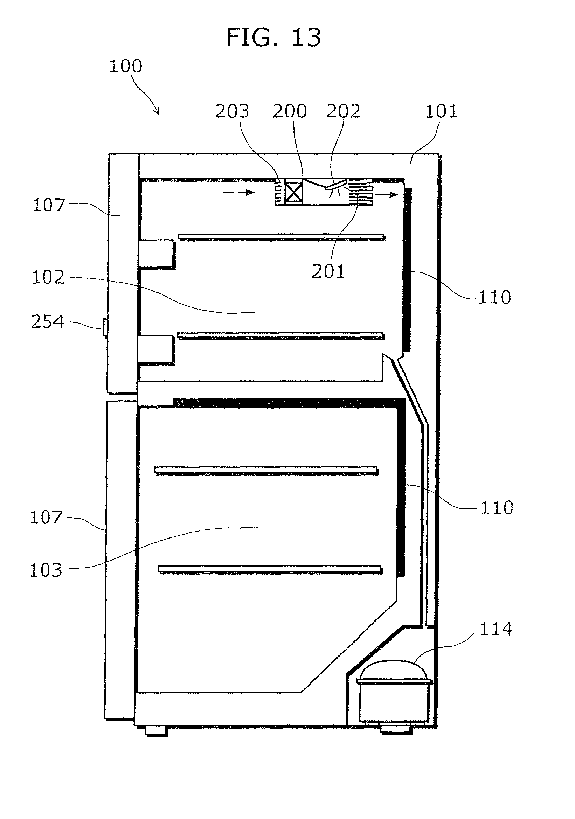

[FIG. 13] FIG. 13 is a longitudinal sectional view of the refrigerator.

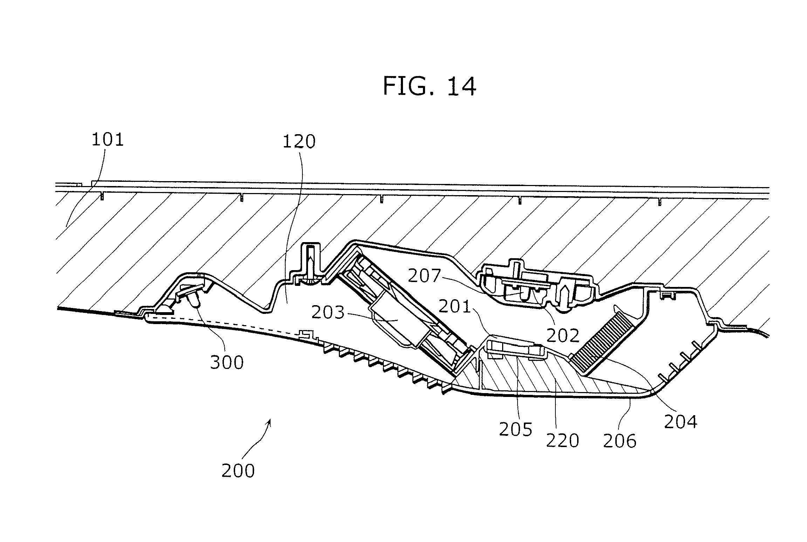

[FIG. 14] FIG. 14 is a cross sectional view showing a sterilization apparatus mounted to the refrigerator.

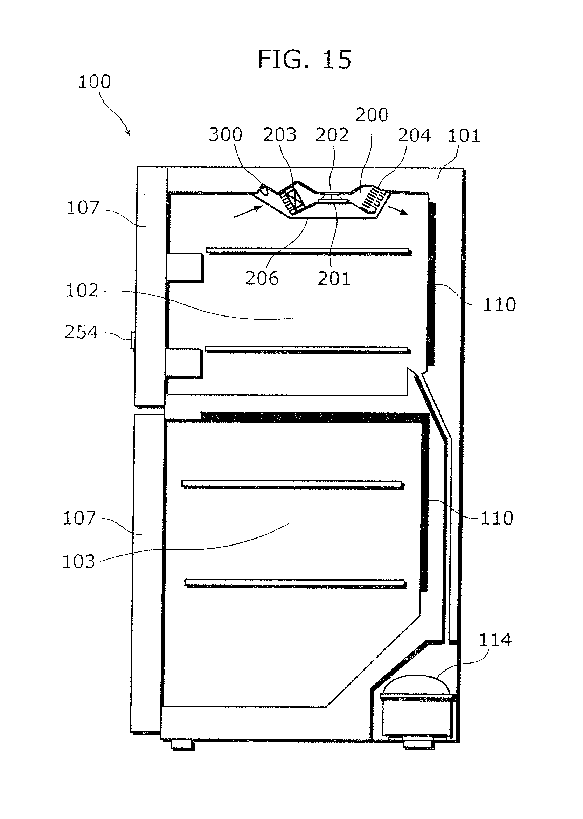

[FIG. 15] FIG. 15 is a longitudinal sectional view schematically showing the refrigerator in the present embodiment.

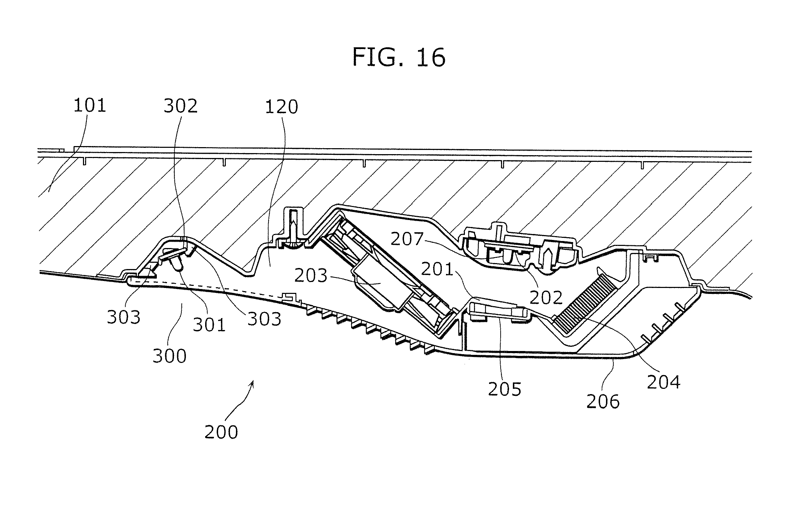

[FIG. 16] FIG. 16 is a cross sectional view of a sterilization apparatus and a lighting unit mounted to the refrigerator.

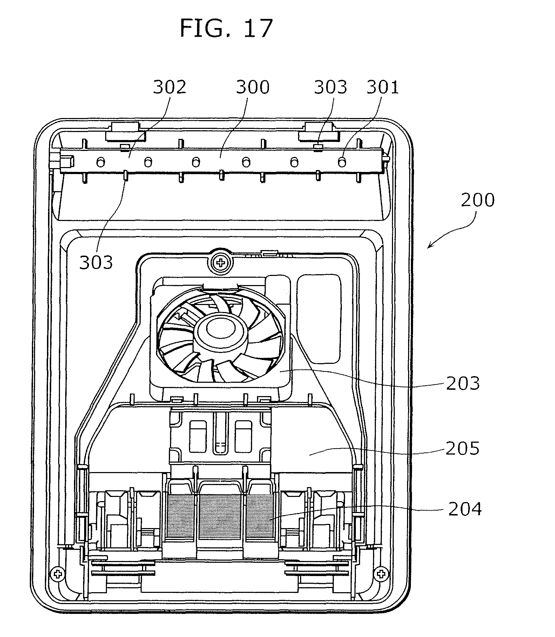

[FIG. 17] FIG. 17 is a bottom view of a sterilization apparatus and a lighting unit mounted to the refrigerator.

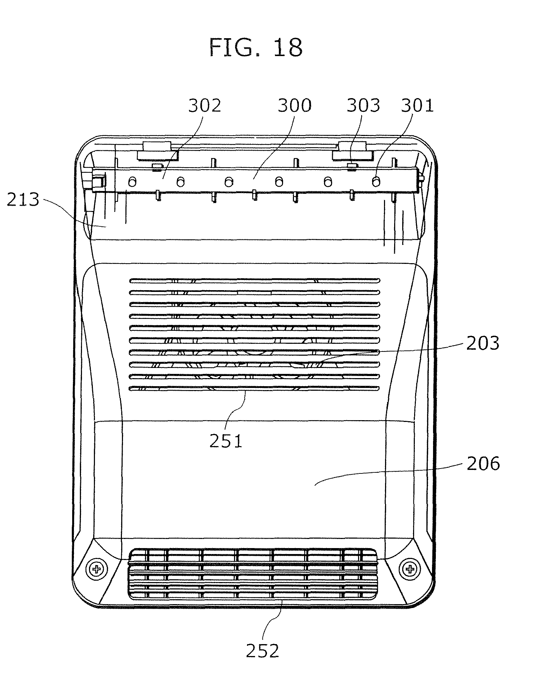

[FIG. 18] FIG. 18 is a base view showing the sterilization apparatus and the lighting unit, which are covered with a cover.

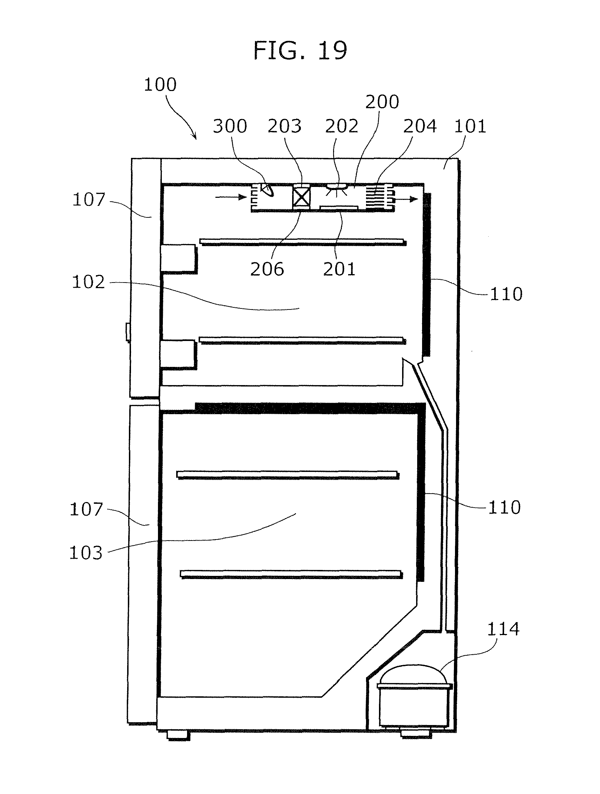

[FIG. 19] FIG. 19 is a longitudinal sectional view of a refrigerator.

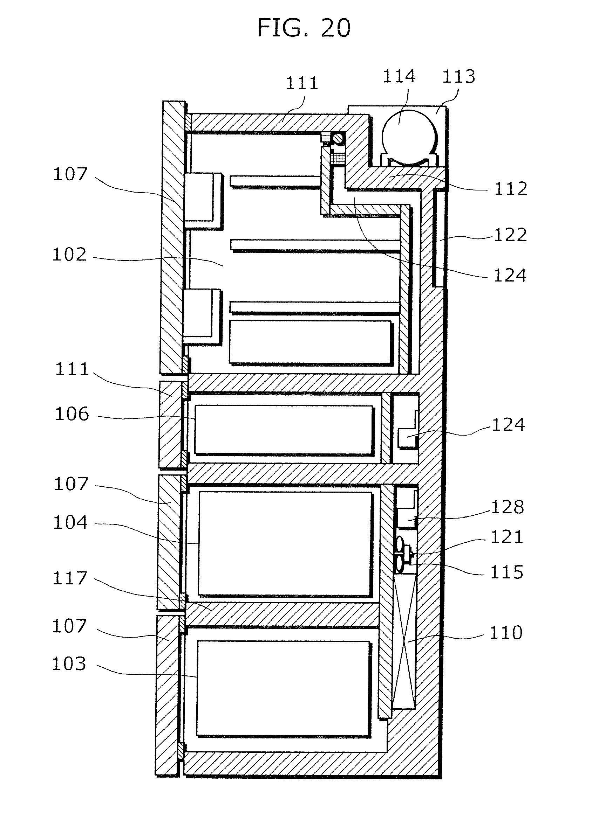

[FIG. 20] FIG. 20 is a longitudinal sectional view of a refrigerator in the present invention.

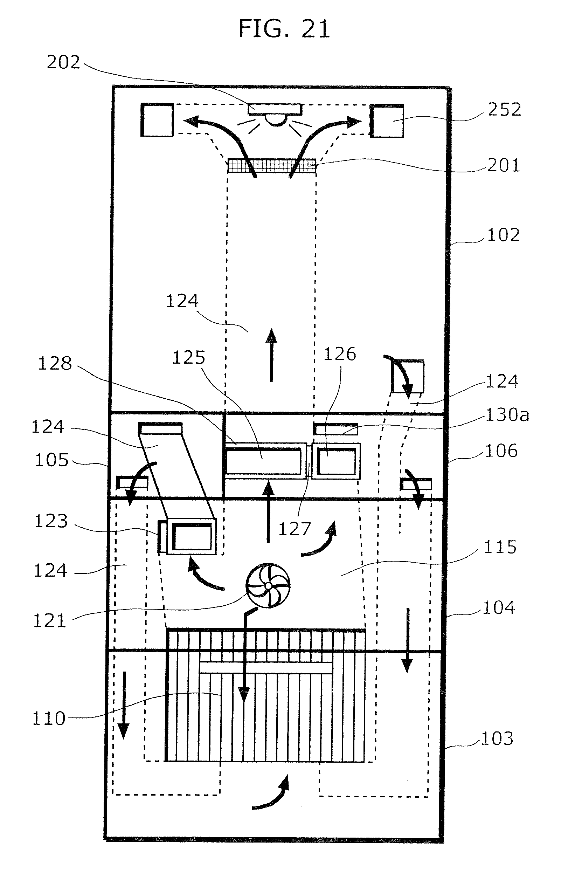

[FIG. 21] FIG. 21 is an elevation view showing a structure of a flow path forming unit of the refrigerator in the present invention.

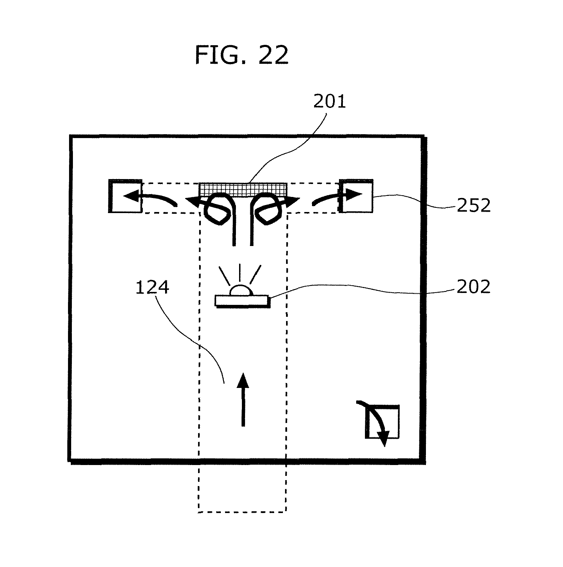

[FIG. 22] FIG. 22 is an elevation view of the refrigerator in the present invention.

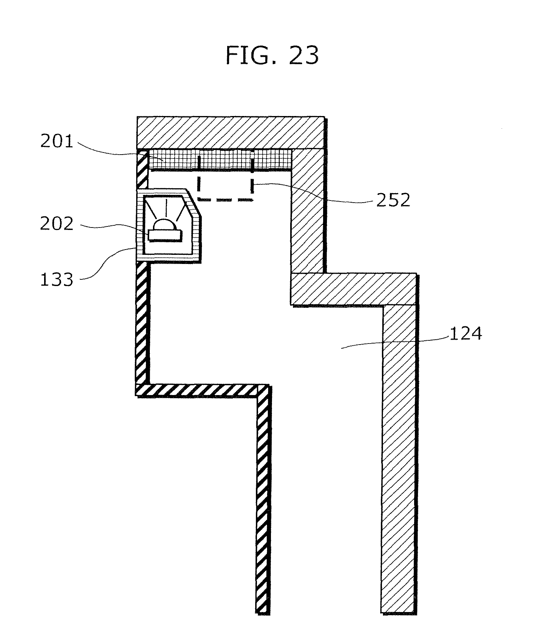

[FIG. 23] FIG. 23 is a longitudinal sectional view of the flow path forming unit including a support object in the present invention.

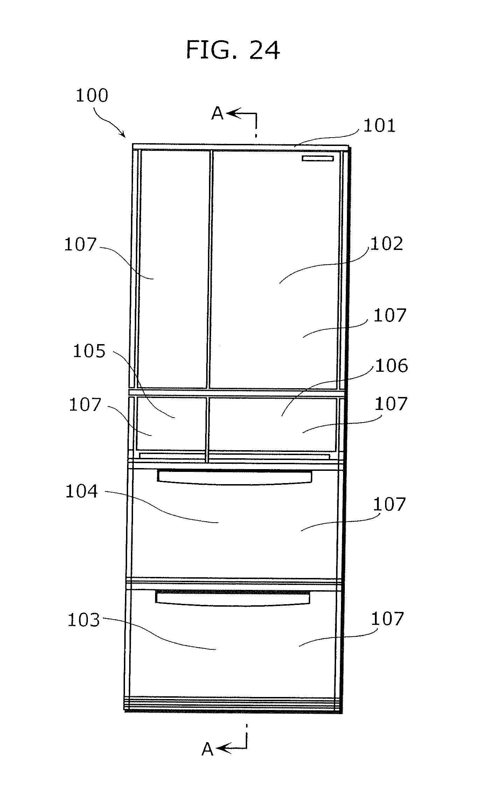

[FIG. 24] FIG. 24 is an elevation view of the refrigerator in an embodiment of the present invention.

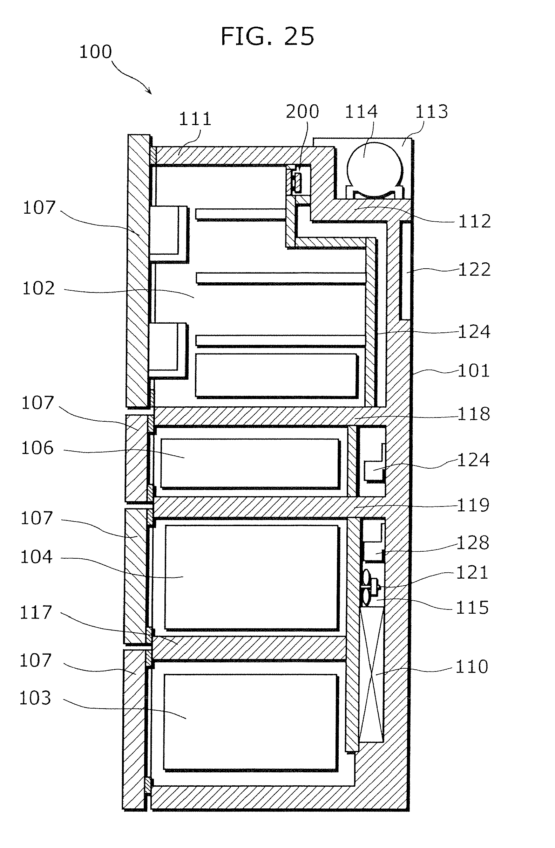

[FIG. 25] FIG. 25 is a longitudinal sectional view of the refrigerator in the present invention.

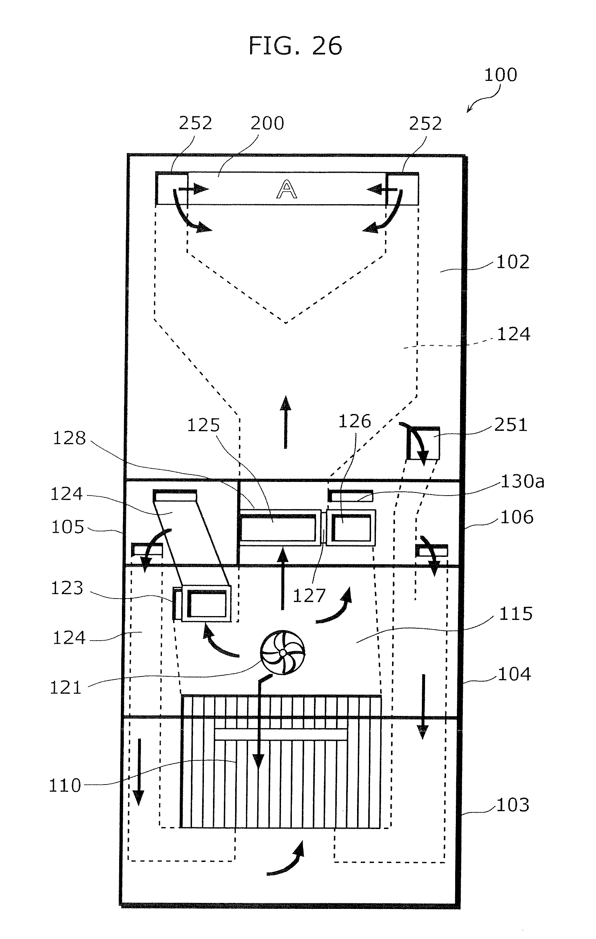

[FIG. 26] FIG. 26 is a diagram showing a structure of the flow path forming unit, included in a circulation path of the cool air in the present embodiment.

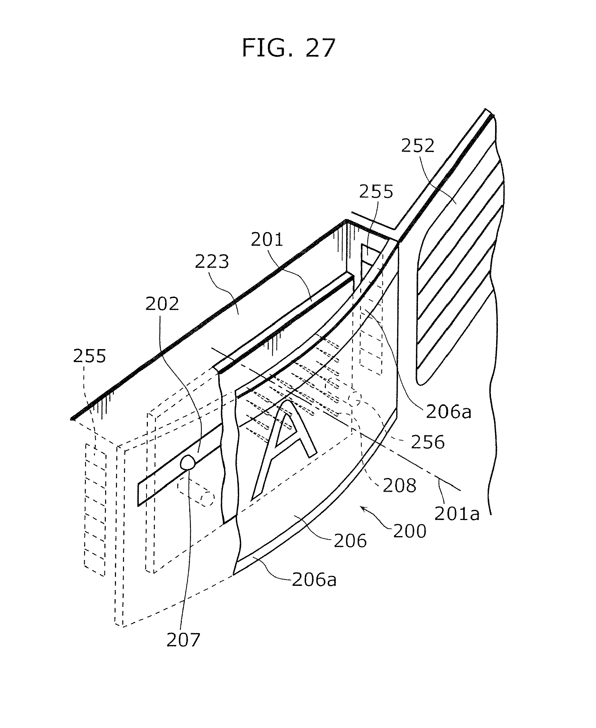

[FIG. 27] FIG. 27 is a perspective view showing a sterilization apparatus mounted to the refrigerator in the present embodiment.

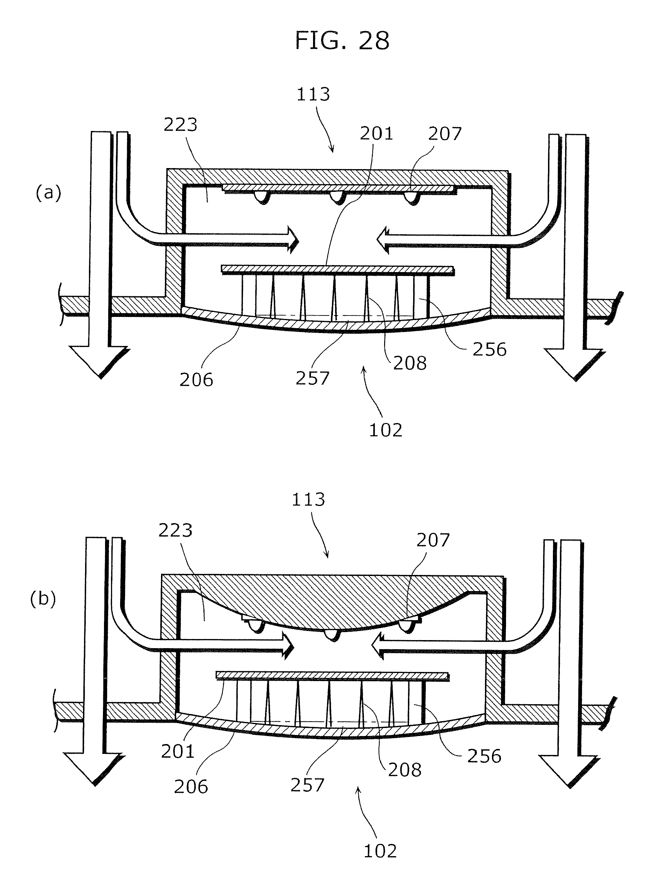

[FIG. 28] FIG. 28 is a cross-sectional view showing an attachment mode of the sterilization apparatus in the present embodiment.

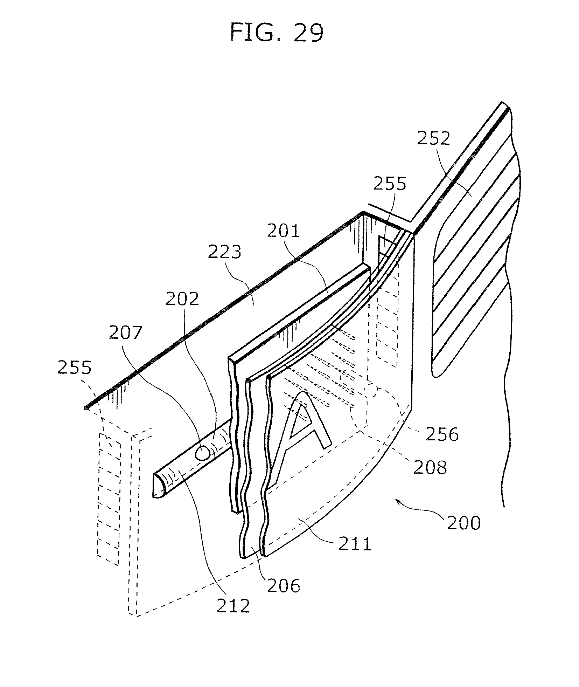

[FIG. 29] FIG. 29 is a perspective view showing another attachment mode of the sterilization apparatus in the present embodiment.

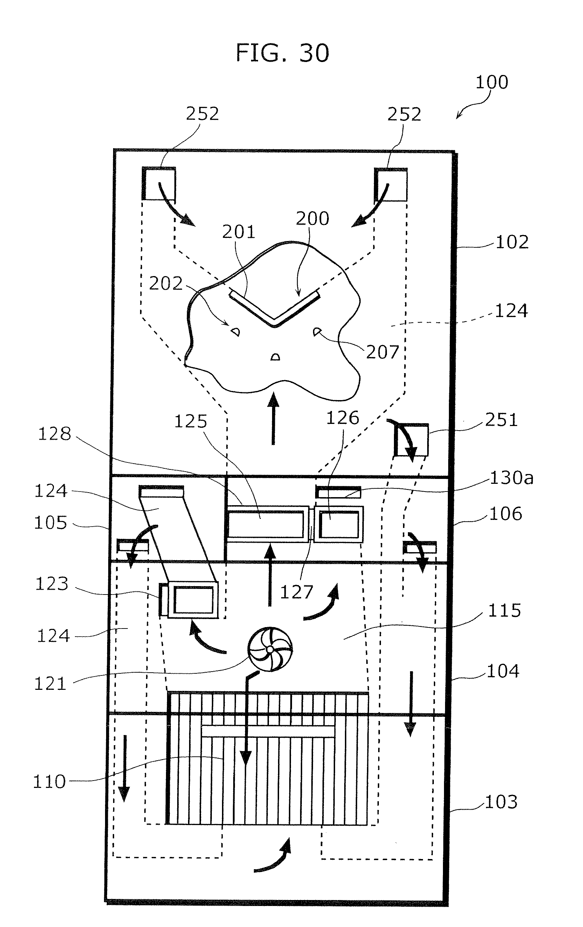

[FIG. 30] FIG. 30 is a diagram showing a sterilization apparatus in an embodiment of the present invention, with a back wall of a refrigerating compartment partially cut away.

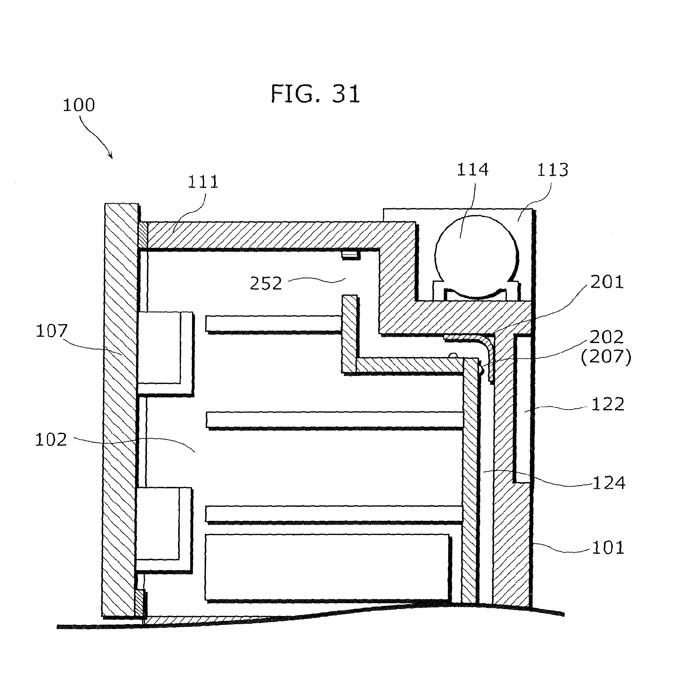

[FIG. 31] FIG. 31 is a cross-sectional view of an upper part of the refrigerator.

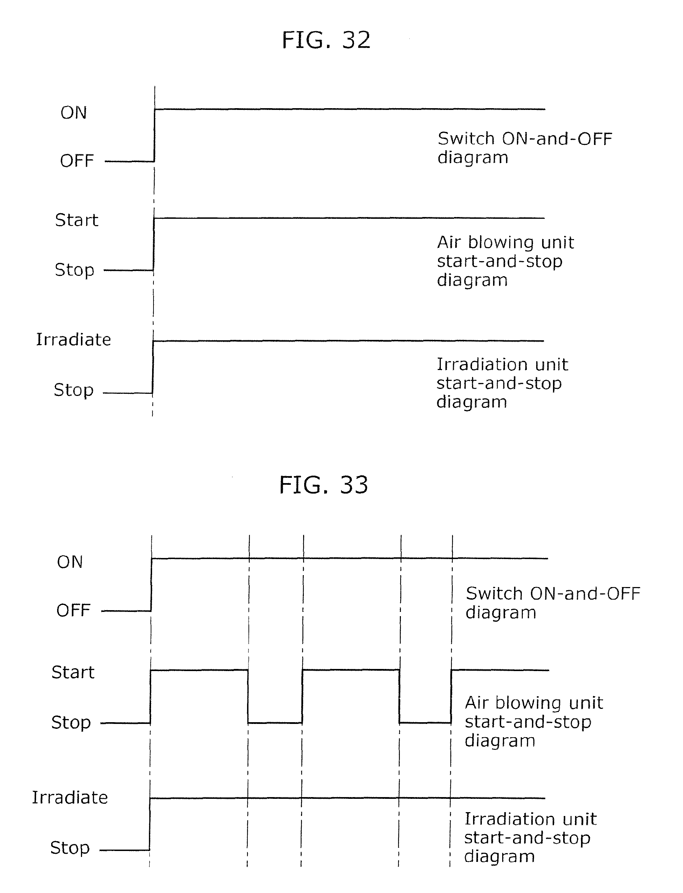

[FIG. 32] FIG. 32 is a control diagram according to the present invention.
[FIG. 33] FIG. 33 is a control diagram according to the present invention.

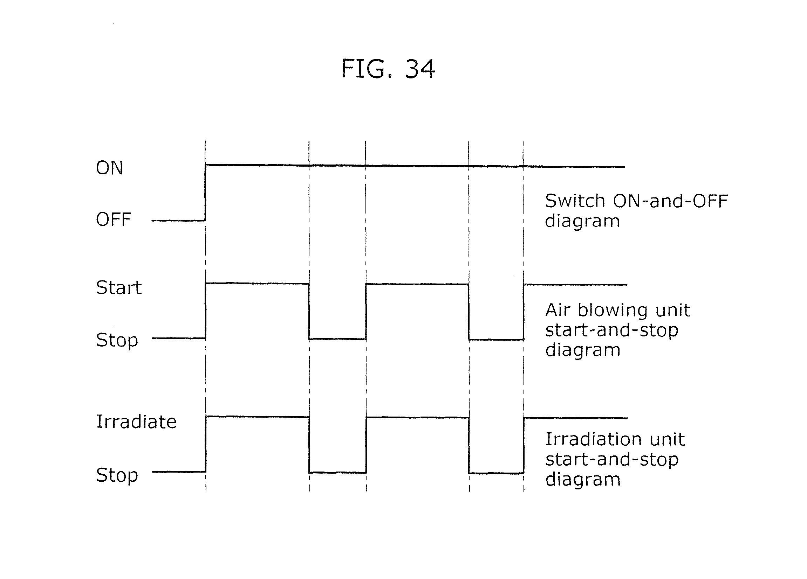

FIG. 34] FIG. 34 is a control diagram according to the present invention.

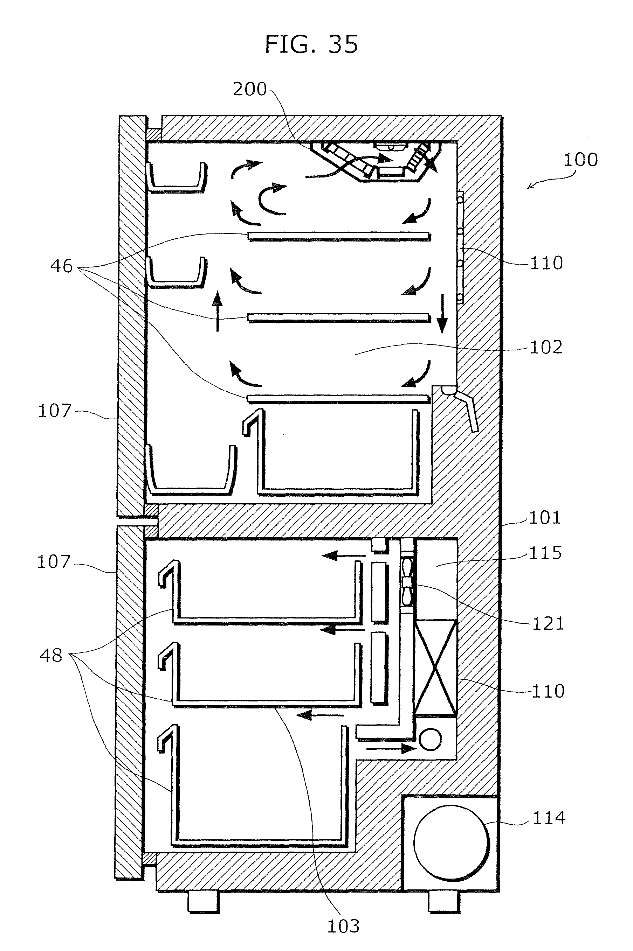

[FIG. 35] FIG. 35 is a center longitudinal sectional view of the refrigerator in an embodiment of the present invention.

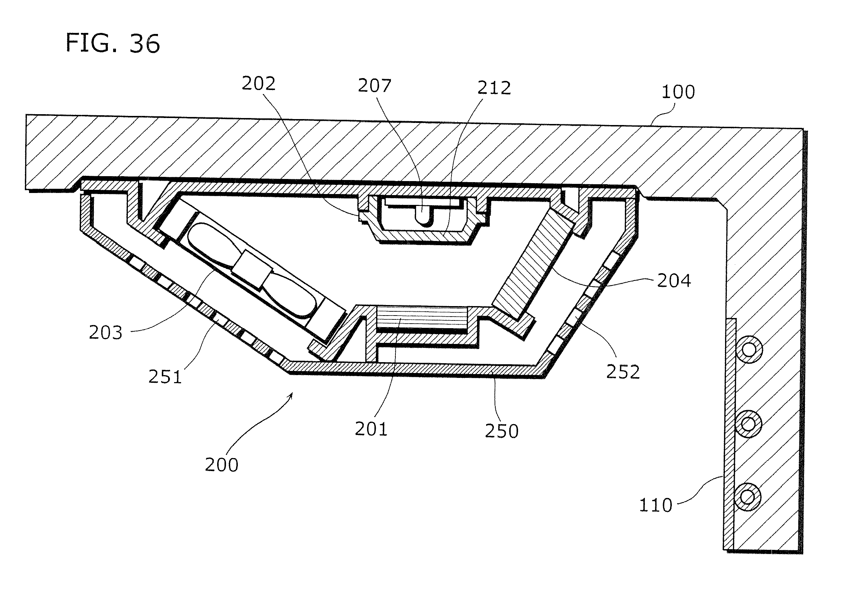

[FIG. 36] FIG. 36 is a center longitudinal sectional view of the sterilization and deodorization apparatus when mounted to the refrigerator in the embodiment of the present invention.

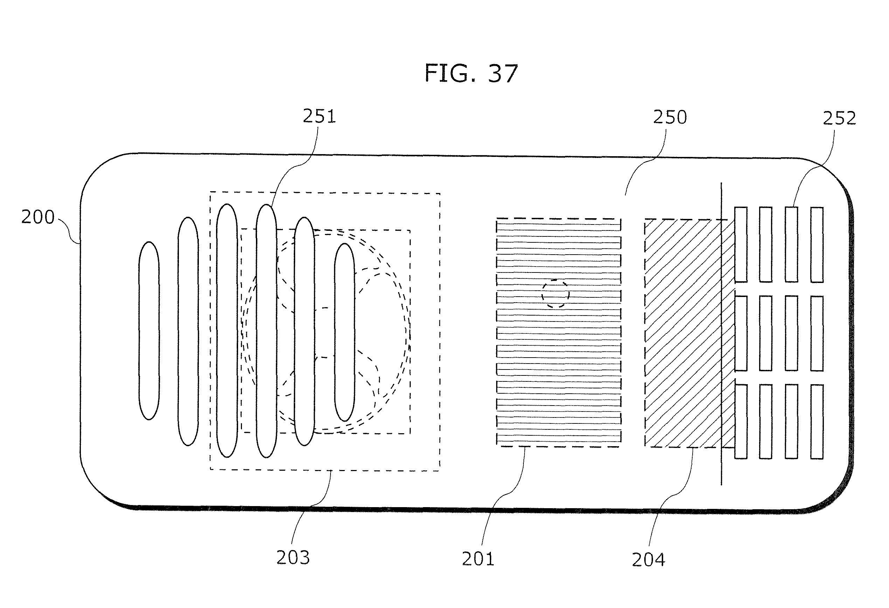

[FIG. 37] FIG. 37 is an outer bottom view of the sterilization and deodorization apparatus mounted to the refrigerator in the embodiment of the present invention.

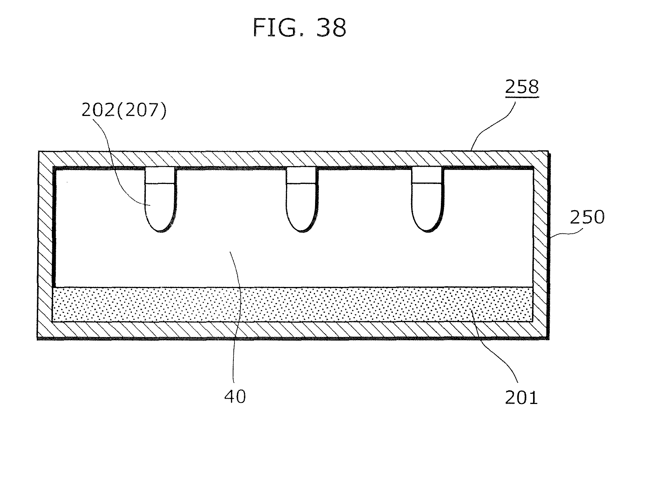

[FIG. 38] FIG. 38 shows a cross-sectional view of an air purifying apparatus which is part of the sterilization apparatus.

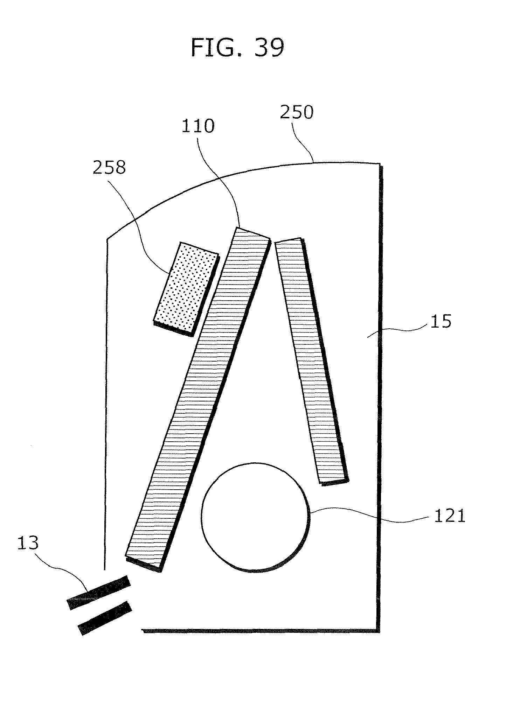

[FIG. 39] FIG. 39 is a cross-sectional view showing indoor equipment of an air conditioner.

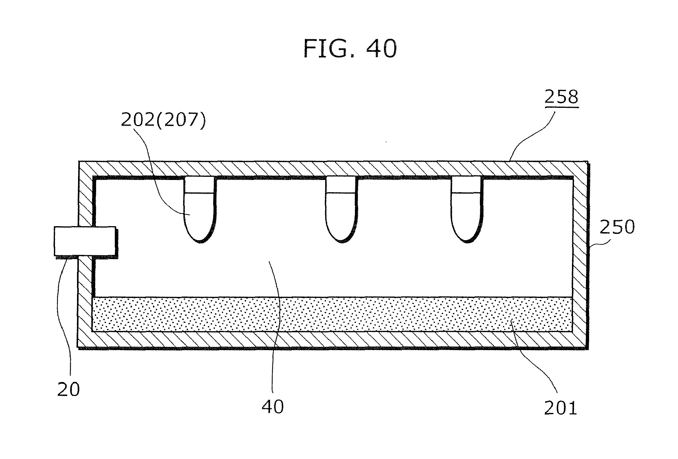

[FIG. 40] FIG. 40 shows a cross-sectional view of the air purifying apparatus in an embodiment of the present invention.

### Numerical References

- 4: Flow Path
- 13: Louver
- 15: Air conditioner
- 20: Supply unit
- 46: Shelf plates
- 48: Case
- 100: Refrigerator
- 101: Heat-insulating main body
- 102: Refrigerating compartment
- 103: Freezing compartment
- 104: Vegetable compartment
- 105: Ice compartment
- 106: Switch compartment
- 107: Heat-insulating door
- 110: Cooler
- 111: First top surface
- 112: Second top surface
- 113: Concavity
- 114: Compressor
- 115: Cooling room
- 120: Concave portion
- 121: Cooling fan
- 122: Control board
- 123: Damper
- 124: Flow path forming unit
- 125: Flap
- 127: Drive unit
- 128: Twin dumper
- 131: Collection opening
- 133: Light-transmissive member
- 200: Sterilization apparatus
- 201: Support
- 201a: Normal line
- 202: Irradiation unit
- 203: Air blowing unit
- 204: Absorption filter
- 206: Cover
- 206a: End edge
- 207: Light source
- 208: Projection
- 209: Narrow portion
- 211: Double cover
- 212: Light source cover
- 213: Transparent window
- 220: Heat-insulating material
- 221: Motor
- 222: Axial-flow fan
- 223: Storage space
- 224: Rotational axis
- 230: Air flow direction
- 240: Air passing direction
- 250: Housing
- 251: Inlet
- 252: Outlet
- 254: Switch
- 255: Branch opening
- 256: Supporting member
- 257: Through hole
- 258: Air purifying apparatus
- 300: Lighting unit
- 301: Light source
- 302: Light board
- 303: Engagement stopper

### Best Mode for Carrying Out the Invention

Next, embodiments of a refrigerator according to the present invention will be described with reference to the drawings.

### (First Embodiment)

FIG. 7 is an elevation view of a refrigerator in a first embodiment of the present invention.

As the figure shows, a refrigerator 100 according to the present embodiment is a refrigerator 100 having French doors, and includes a storage compartment divided into upper and lower parts in a heat-insulating main body 101.

The storage compartment, which is divided into upper and lower parts in the refrigerator 100, is referred to as a refrigerating compartment 102 and a freezing compartment 103, respectively, according to the functions (cooling temperature) thereof.

At the front opening of the refrigerating compartment 102, a rotational heat-insulating door 107 filled with foam insulation such as urethane is provided. Likewise, the heat-insulating door 107 is also provided at the front of the freezing compartment 103. These doors conceal the storage compartment to allow opening and closing in such a manner as to prevent leakage of cool air.

The refrigerating compartment 102 is a storage compartment in which the temperature is maintained low enough for chilled storage but not too low for freezing. Specifically, a specific temperature of the lower limit is normally set to 1 to 5 °C.

The freezing compartment 103 is a storage compartment set to a freezing temperature zone. Specifically, the temperature is normally set to - 22 to - 18 °C for frozen storage, but it is also set to a lower temperature of - 30 or 25 °C, for example, for improving the frozen-storage state.

FIG. 8 is a longitudinal sectional view schematically showing the refrigerator in the present embodiment.

As the figure shows, the refrigerator 100 internally includes a sterilization apparatus 200 and a cooler 110.

The heat-insulating main body 101 is a box-shaped body filled with a heat-insulating material, for example, rigid foamed urethane between an outer case and an inner case. The heat-insulating main body 101 insulates the inside of the heat-insulating main body 101 from the periphery.

Inside the heat-insulating main body 101, a cooler 110 as a cooling unit is embedded in the heat-insulating main body 101.

The cooler 110 is a member which forms a cooling cycle along with a compressor 114 and directly cools the inside of the heat-insulating main body 101 by drawing heat away from the air inside the heat-insulating main body 101 through evaporation of the refrigerant inserted into the cooler 110.

Next, the sterilization apparatus 200 will be described.

FIG. 9 is a cross sectional view of a sterilization apparatus mounted to the refrigerator.

FIG. 10 is a bottom view of a sterilization apparatus mounted to the refrigerator.

The sterilization apparatus 200 of the present embodiment is an apparatus which can forcibly sterilize the bacteria and spores that are present in the air inside the heat-insulating main body 101 and can also achieve deodorization by decomposing an organic substance that is present in the air. The sterilization apparatus 200 includes: a support 201 which is a photocatalyst support; an irradiation unit 202 which irradiates the support 201 with an excitation ray which excites the photocatalyst; an air blowing unit 203; an absorption filter 204; a flow path forming unit 124, and a cover 206.

The sterilization apparatus 200 is attached to a concave portion 120 provided in the heat-insulating main body 101. In addition, a cover 206 is attached so as to cover the sterilization apparatus 200 which is attached to the heat-insulating main body 101. A predetermined space is provided between the sterilization apparatus 200 and the cover 206.

The air blowing unit 203 is placed closest to the heat-insulating door 107 of the sterilization apparatus 200. In addition, the air blowing unit 203 blows the air into a direction indicated by arrows in the figure.

The absorption filter 204 is placed closest to the cooler 110 in the sterilization apparatus 200.

The support 201 is placed between the air blowing unit 203 and the absorption filter 204.

In addition, the support 201, the irradiation unit 202, the air blowing unit 203, and the absorption filter 204 are attached to the ceiling of the heat-insulating main body 101.

The support 201 is a porous resin member which allows increased contact with the air, and also is a filter member made of fibers into which a photocatalyst is kneaded. In addition, used for the base-material resin is a resin which transmits light having a wavelength that easily excites the photocatalyst.

The photocatalyst used for the support 201 is a catalyst which can sterilize the bacteria in the air and deodorize odor components (such as an organic substance) in the air by irradiation of light having a given wavelength, and is considered as a substance which activates the component in the air (for example, ionization or radicalization) and can thereby perform sterilization and deodorization. Specifically, silver oxide or titanium oxide can be given as an example of the photocatalyst.

To fulfill its sterilization and other functions, silver oxide requires light having a visible blue-color wavelength range approximately 400 nm to 580 nm. On the other hand, light having a wavelength 380 nm is necessary for titanium oxide to fulfill its sterilization and other functions.

The irradiation unit 202 is an apparatus including: a light source 207 which can emit light having a wavelength that can excite the photocatalyst, and a circuit board (not shown) for lighting the light source 207.

It is sufficient that the light source 207 can emit a predetermined amount of light having wavelengths including the wavelength described above, and an ultraviolet lamp or an ordinary electric bulb can be given as an example. In addition, when the photocatalyst is silver oxide, it is possible to achieve longer life and reduced cost by adopting an LED, which emits light in blue (470 nm) of a visible light range. In addition, when the photocatalyst is silver titanium, it is also possible to adopt a UV-LED which emits an ultraviolet ray of 380 nm.

In the present embodiment, silver oxide is used as the photocatalyst, and three LEDs lined up on a strip of board are used as the light source 207 for the irradiation unit 202.

The air blowing unit 203 includes a motor 221, an axial-flow fan 222, and a casing in which to hold these units. When the motor 221 rotates by power activation, the air blowing unit 203 has a function to blow cool air in a predetermined direction (direction indicated by arrows in FIG. 9) by the axial-flow fan 222 directly connected to a rotational axis of the motor 221. In other words, the motor 221 is provided in the center of the air blowing unit 203 and formed with an axial-flow fan 222 of which unidirectionally-bent blades are attached radially to the rotational axis of the motor 221, and the motor 221 and the axial-flow fan 222 are housed in one housing. Then, when the motor 221 rotates by power activation, the air blowing unit 203 has a function to blow air unidirectionally, using the axial-flow fan 222 driven by the motor 221.

The axial-flow fan 222 is placed such that a rotational axis 224 of the axial-flow fan 222 intersects with an air flow direction 230. That is, the air blowing unit 203 is placed almost flat with respect to a ceiling surface of the heat-insulating main body 101.

The absorption filter 204 has a function as an absorption filter which absorbs and removes odor components that are present inside the heat-insulating main body 101. Specifically, a honeycomb-structured member or the like made of activated carbon can be given as an example of the absorption filter 204. Such an absorption filter 204 can absorb the odor components present in the air by letting the air through the pores of the honeycomb structure.

The absorption filter 204 is placed such that an air passing direction 240 of the absorption filter 204 intersects with the air flow direction 230. Accordingly, as in the case of the air blowing unit 203, the absorption filter 204 is placed almost flat with respect to the heat-insulating main body 101.

The flow path forming unit 124 is a member made of resin, and can hold the support 201, the air blowing unit 203, and the absorption filter 204. The sterilization apparatus 200 is formed inside the heat-insulating main body 101 by threadably mounting, to a predetermined position in the heat-insulating main body 101, the flow path forming unit 124 to which the support 201, the air blowing unit 203, and the absorption filter 204 are attached.

In addition, a duct for inserting further cool air is provided between the flow path forming unit 124 and an inner wall of the heat-insulating main body 101. Accordingly, the irradiation unit 202, the air blowing unit 203, and the absorption filter 204 are provided inside the duct, and these make up the sterilization apparatus 200.

The flow path forming unit 124, when mounted to the heat-insulating main body 101, has a structure that does not cause leakage (is unlikely to cause leakage) of the air from the flow path forming unit 124, except for a portion through which the air is introduced into the flow path forming unit 124 and a portion through which the introduced air is derived from the flow path forming unit 124. Accordingly, a flow path for the air blown by the air blowing unit 203 is formed between the heat-insulating main body 101 and the flow path forming unit 124.

The flow path forming unit 124 holds a support 201 such that a largest face of the support 201 faces the irradiation unit 202 attached on the side of the heat-insulating main body 101.

The flow path forming unit 124 holds the support 201 detached from the flow path forming unit 124 so that the support 201 does not directly contact the flow path forming unit 124. Specifically, the support 201 is attached to a tip of each of thin column-shaped members extending from the flow path forming unit 124. With this, the support 201 is almost floating in the air, thus allowing almost all the area of the support 201 to be exposed to the air introduced by the air blowing unit 203. In addition, by providing the support in a detached condition from the flow path forming unit 124, dew condensation generated in the flow path forming unit 124 is prevented as much as possible from moving onto the support 201. In addition, since the support 201 has its surface held upright so as not to flap in the flowing air or not to bend due to gravity, no shadow portion out of reach of the light from the irradiation unit 202 is generated, thus enabling efficient excitation of the photocatalyst that is supported on the support 201.

The flow path forming unit 124 is held almost flat with respect to a face of the heat-insulating main body, with the air blowing unit 203 and the absorption filter 204 attached to the heat-insulating main body 101. By thus holding the air blowing unit 203 and the absorption filter 204 almost flat with respect to a horizontal direction, it is possible to reduce as much projection as possible of the sterilization apparatus 200 into the heat-insulating main body 101, to introduce a large amount of air into the sterilization apparatus 200, and to let the large amount of air flow through the absorption filter 204 having a large surface area.

The flow path forming unit 124 has, in an intermediate part of the flow path for the air, a narrow portion 209 having a cross-sectional area narrower than in other portions. The narrow portion 209 is a throat formed from a shape of the concave portion 120 of the heat-insulating main body and a shape of the flow path forming unit 124, and has a function to narrow the air flow.

The irradiation unit 202 is mounted at the bottom of the concave portion 120 in the heat-insulating main body 101. The portion forms the narrow portion 209, projecting such that the distance between the support 201 and the irradiation unit 202 is shorter.

Note that in the present embodiment, the narrow portion 209 is formed from the concave portion 120 and the flow path forming unit 124 but is not limited to this. Even when the bottom of the concave portion 120 is flat without any projection, the portion is also considered as the narrow portion 209 as long as the flow path forming unit 124 is projecting because the projection narrows the flow path.

With the narrow portion 209 described above, the density of the flowing cool air increases, so that more cool air contacts or passes near the support 201. Accordingly, it is possible to perform efficient sterilization.

In a portion mounted with the sterilization apparatus 200 in the heart-insulating main body 101, the concave portion 120 that is concavity is formed where the heat-insulating material is thinner than in other portions.

By mounting the sterilization apparatus 200 to the concave portion 120, it is possible to avoid projection of the sterilization apparatus 200 into the heat-insulating main body 101, thereby suppressing as much decrease possible in internal capacity of the heat-insulating main body 101. In addition, the thinner heat-insulating material allows the temperature of the sterilization apparatus 200 to rise higher than in the other portions, and thus making it possible to perform efficient sterilization. In addition, it is possible to reduce, as much as possible, dew condensation generated inside the sterilization apparatus 200.

FIG. 11 is a bottom view showing the sterilization apparatus and a lighting unit which are covered with a cover.

The cover 206 is a plate-shaped member which can cover the whole sterilization apparatus 200 attached to the heat-insulating main body 101, and includes an inlet 251 and an outlet 252. In addition, louvers for controlling the air flow are attached to the inlet 251 and the outlet 252. The cover 206 improves design versatility by covering over the sterilization apparatus 200, and also insulates the sterilization apparatus 200 from the cool air inside the heat-insulating main body 101 by being provided at a predetermined distance from the sterilization apparatus 200. Accordingly, the cover 206 protects the sterilization apparatus 200 from dew condensation, thus preventing performance degradation of the sterilization apparatus 200. In addition, the cover 206 covers a lighting unit 300 embedded in the heat-insulating main body 101.

In the cover 206, a portion opposite to the lighting unit 300 is transparent and the other portions are semi-transparent like frosted glass, and the entire flow path forming unit 124 is semi-transparent like frosted glass. This allows part of the visible light emitted from the light source 207 and the reflected light of the visible light reflected within the sterilization apparatus 200 to leak into the heat-insulating main body 101. With this, it is possible to check that the sterilization is actually performed, and to recognize from outside whether or not the irradiation unit 202 is in trouble.

The operation and effects of the refrigerator 100 thus configured will be described below.

The air, which contains odor components (such as an organic substance) or bacteria that is present inside the heat-insulating main body 101, passes through the inlet 251 in the cover 206 by the blowing force of the air blowing unit 203, and is introduced into the sterilization apparatus 200 via the air blowing unit 203.

Here, the temperature of the air introduced into the sterilization apparatus 200 rises to a temperature appropriate for deodorization and sterilization due to the heat such as the heat passing through the concave portion 120 having a thinner heat-insulating material and provided in the heat insulating main body 101, the heat generated in the irradiation unit 202, and the heat generated in the lighting unit 300. In addition, even when the heat-insulating door 107 is open and the external air invades the heat-insulating main body 101 to be led into the sterilization apparatus 200, the temperature of the whole sterilization apparatus 200 is high enough not to cause dew condensation.

The air in such a state as described above passes licking a face having a largest area of the support 201. Here, since the narrow portion 209, which is sandwiched between the irradiation unit 202 and the flow path forming unit 124 that is placed opposite to the irradiation unit 202, is set narrower than other portions, the flowing cool air has high density in the narrow portion 209 and as such contacts the support 201 efficiently. Accordingly, the odor components and bacteria contained in the cool air are efficiently captured on the surface of the support 201. The captured odor components and bacteria are deodorized and sterilized by oxidative decomposition caused by ordinary catalytic action (not by photocatalytic action) and the sterilization effect of the silver oxide. Also in this stage, it can be considered that the narrow portion 209 allows efficient sterilization and deodorization. With this, since odor-decomposition and sterilization effects are produced by the silver-oxide action even when not irradiated with light, it is possible to reduce the volume of irradiance and the amount of time for the irradiation while, at the same time, maintaining the desired deodorization and sterilization effects, thus achieving longer life and increased energy-saving effects of the irradiation unit. Furthermore, with the light energy (blue light or an ultraviolet ray) irradiated from the light source 207, the silver oxide having an absorption spectrum in these wavelength ranges is excited by the blue light energy, so that the photocatalyst on the surface of the support 201 is excited. When the photocatalyst is excited, hydroxyl (OH) radical is generated from moisture in the air, so that oxidative decomposition is performed on the odor components captured on the support 201 or present near the support 201 and the bacteria are lysed. In this stage, it can be considered that more OH radial is generated by the effect produced by the narrow portion 209, so that the decomposition of organic substances and the lysis of the bacteria are promoted.

With the catalytic action described above, the air to be sterilized and deodorized is cool air having a highest temperature within the heat-insulating main body 101. Accordingly, it can be considered that the highest sterilization and deodorization efficiency is obtained.

Furthermore, odor components which are not fully decomposed on the surface of the support 201 pass, along with air, through the pores of the absorption filter 204 having a honeycomb structure, and are absorbed and held by the absorption filter 204.

As described above, the air passing though the sterilization apparatus 200 becomes clean air that is deodorized and sterilized, and is blown into the heat-insulating main body 101 from the outlet 252 provided in the cover 206. Accordingly, by continuing the sterilization, it is possible to decrease the number of bacteria and reduce odor components.

The air which is blown out is blown toward the cooler 110 and cooled to a temperature appropriate for storing food and so on. Accordingly, even when the temperature inside the sterilization apparatus 200 rises, the air is cooled down after blown into the heat-insulating main body 101, thus giving no negative influence on the food and so on in storage.

On the other hand, inside the heat-insulating main body 101, overall air convection is promoted by introducing air into the sterilization apparatus 200 and deriving air from the sterilization apparatus 200. Accordingly, it is possible to prevent the cool air from staying at the bottom of the heat-insulating main body 101, thus making it possible to even out the temperature inside the heat-insulating main body 101. Furthermore, the OH radical generated by the sterilization apparatus 200 convectively flows along with the air in the heat-insulating main body 101, and performs deodorization and sterilization also in the heat-insulating main body 101.

Furthermore, the air cooled by the cooler 110 provided in the back wall of the heat-insulating main body 101 flows down to the bottom of the heat-insulating main body 101 due to its high specific gravity. Accordingly, the air whose temperature has risen after passing by the stored food and the heat-insulating door 107 flows up toward a portion surrounded by the ceiling of the heat-insulating main body 101 and the heat-insulating door 107. The air blowing unit 203 blows air in a direction that promotes convective flow, without going against the above-described very moderate convective flow generated in the heat-insulating main body 101. As a result, a strong convective flow is forcibly generated in the heat-insulating main body 101.

Accordingly, it is possible to prevent the cool air from staying at the bottom of the heat-insulating main body 101, thus making it possible to even out the temperature inside the heat-insulating main body 101. Furthermore, the OH radical generated by the sterilization apparatus 200 convectively flows along with the air in the heat-insulating main body 101 and performs deodorization and sterilization also in the heat-insulating main body 101.

As described above, the refrigerator can physically perform sterilization and deodorization using the sterilization apparatus 200, without using an organic sterilization agent or the like. Thus, it is no longer necessary to consider the influences of organic sterilization agents over a human body and so on. In addition, since the photocatalyst can perform sterilization and deodorization without changing itself, it is possible to maintain its effects semipermanently.

Note that a face having a largest area of the support 201 may be held parallel to a vertical face. By thus providing the support 201, even in the case of dew condensation on the support 201, the water condensed on the face having the largest area is pulled downward due to gravity, so that the face having the largest area is drained. Accordingly, it is possible to sterilize and deodorize the branched air by using a photocatalyst, without being interrupted by such dew condensation water.

In addition, in the embodiment described above, the irradiation unit 202 is directly attached to the heat-insulating main body 101, and the flow path is formed with the heat-insulating main body 101 and the flow path forming unit 124. However, the present invention is not limited to this.

For example, as FIG. 12 shows, the sterilization apparatus may be a unit-type sterilization apparatus which is detachable from the heat-insulating main body 101 and in which the irradiation unit 202, the support 201, and the air blowing unit 203 are attached to the box-shaped flow path forming unit 124 that is a rectangular duct. This configuration makes it easier to attach or detach the sterilization apparatus to or from the refrigerator. Furthermore, the lighting unit 300 may be provided in the unit-type sterilization apparatus. Especially, when the light source 207 and the lighting unit 300 are both LEDs, it is possible to share the power circuit, thus facilitating the assembly of the refrigerator.

In addition, in the embodiment described above, the absorption filter 204 and the support 201 are assumed as separate members, but the present invention is not limited to this. For example, as shown in FIG. 13, the support 201 on which the photocatalyst is supported may have an absorption function that can hold the odor. In other words, the photocatalyst may be supported onto the absorption filter 204 which absorbs and holds the odor. Note that it is preferable to obliquely irradiate the support 201 having the absorption function, so that the air flow is not disturbed.

In the manner described above, it is possible to realize downsizing of the sterilization apparatus 200, and to suppress the reduction of the capacity inside the heat-insulating main body 101. It is further possible to achieve cost reduction.

As described above, a refrigerator according to the present invention includes a heat-insulating main body 101 which is made of a heat-insulating material and in which a storage compartment is formed, a heat-insulating door 107 as a door attached to an opening provided on the heat-insulating main body 101 so as to be openable and closable, and a cooling unit which directly cools the air inside the heat-insulating main body 101, and the refrigerator includes: a support 201 which is placed in the storage compartment and on which the photocatalyst is supported; an irradiation unit 202 which irradiates the support 201 with excitation light which excites the photocatalyst; and an air blowing unit 203 which forcibly blows the air inside the storage compartment toward the support 201. Thereby, since sterilization and deodorization is performed using the photocatalyst in the air flow generated by the air blowing unit 203, it is possible to cause a convection current of sterilized and deodorized clean air in the storage compartment. Accordingly, it is possible to keep the whole storage compartment clean and preserve the food longer. Furthermore, since the air blown by the air blowing unit 203 causes a forcible convection in the storage compartment, it is possible to remove temperature irregularity inside the storage compartment.

Thus, the refrigerator according to the present invention forcibly blows the air inside the storage compartment, sterilizes bacteria and so on contained in the air, and blows the sterilized air into the storage compartment. Accordingly, it is possible to sterilize the inside of the storage compartment as a whole by forcing convection of the air inside the storage compartment.

Furthermore, it is preferable that the flow path forming unit 124 which forms a flow path for the air blown by the air blowing unit 203 be included and that the support 201 be provided in the flow path formed by the flow path forming unit 124.

With this, the support 201 can be exposed to as much air as possible, thus increasing the photocatalytic effect so as to improve sterilization and deodorization efficiencies.

Furthermore, it is preferable that the absorption filter 204 which absorbs and holds odors in the refrigerator be included, and that the absorption filter 204 be placed downstream from the support 201 in the flow path.

With this, it is possible to enhance the deodorization effect and so on, and furthermore, by simply providing the absorption filter downstream from the support 201, it becomes only necessary to hold the remaining odors after the photocatalytic process, thereby extending the life of the absorption filter.

In addition, the support 201 may have an absorption function to absorb and hold the odor in the storage compartment.

With this, since the support 201 can absorb and hold the odor in addition to the sterilization and deodorization effects of the photocatalyst, it is possible to provide a compact and highly-effective support 201. Accordingly, it is possible to keep the storage compartment in a hygienic condition without scarifying the capacity of the storage compartment.

In addition, a sterilization apparatus according to the present invention is attached to a refrigerator including a door attached, so as to be openable and closable, to an opening provided on a heat-insulating main body 101 which is made of a heat-insulating material and in which a storage compartment is formed, and a cooling unit which directly cools air inside the heat-insulating main body 101, and the sterilization apparatus includes: a support 201 on which a photocatalyst is supported; an irradiation unit 202 which irradiates the support 201 with excitation light which excites the photocatalyst; an air blowing unit 203 which forcibly blows the air inside the storage compartment toward the support 201; and a flow path forming unit 124 which forms a flow path for the air blown by the air blowing unit 203, and the support 201, the irradiation unit 202, and the air blowing unit 203 are attached to the flow path forming unit 124.

This makes it easier to attach or detach the sterilization apparatus to or from the refrigerator.

In addition, in the embodiment described above, a space is formed between the sterilization apparatus 200 and the cover 206, but the present invention is not limited to this. For example, as shown in FIG. 14, a heat-insulating member 220 may be provided between the sterilization apparatus 200 and the cover 206. This heat-insulating member 220 thermally and steadily insulates the sterilization apparatus 200 from the inside of the heat-insulating main body 101, thereby making it possible to enhance the sterilization and deodorization functions and also to prevent generation of dew condensation in the sterilization apparatus 200 as much as possible.

As described above, a refrigerator according to the present invention includes a heat-insulating main body 101 which is made of a heat-insulating material and in which a storage compartment is formed, a door attached to an opening provided on the heat-insulating main body 101 so as to be openable and closable, and a cooler 110 that is a cooling unit which directly cools the air inside the heat-insulating main body 101, and a sterilization apparatus 200 which sterilizes and deodorizes an inside of the storage compartment, and the sterilization apparatus 200 is placed in the storage compartment and includes: a support 201 which is placed in the storage compartment and on which a photocatalyst is supported; an irradiation unit which irradiates the support 201 with excitation light which excites the photocatalyst; an air blowing unit 203 which forcibly blows the air inside the storage compartment toward the support 201; and a flow path forming unit 124 which forms a flow path for the air blown by the air blowing unit 203, and the refrigerator includes a cover 206 attached to an inner wall of the heat-insulating main body 101 and covering the sterilization apparatus 200.

With this, it is possible to isolate the temperature within the sterilization apparatus from the temperature inside the storage compartment, thereby making it possible to maintain the sterilization apparatus at a temperature more appropriate for photocatalysis. Accordingly, for example, even when humid air is introduced into the storage compartment when opening or closing the door of the refrigerator, it is possible to prevent generation of dew condensation on the support 201.

Furthermore, it is preferable to include a heat-insulating member, which is to be placed between the sterilization apparatus and the cover.

With this, it is possible to further increase the effects described above.

In addition, it is preferable that the heat-insulating main body 101 include a concave portion that opens toward the storage compartment, and that the sterilization apparatus be attached so as to be embedded in the concave portion.

This facilitates introduction of the heat outside the refrigerator into the sterilization apparatus, thus making it easier to maintain the temperature of the sterilization apparatus at a temperature appropriate for photocatalysis. Accordingly, it becomes easier to suppress the generation of dew condensation.

The refrigerator according to the present invention thermally isolates the sterilization apparatus from the inside of the storage compartment. Accordingly, it becomes possible to maintain the interior temperature of the sterilization apparatus at a temperature appropriate for photocatalysis, and also to prevent generation of dew condensation in the sterilization apparatus.

Thus, a refrigerator according to the present invention includes a heat-insulating main body 101 which is made of a heat-insulating material and in which a storage compartment is formed, a door attached to an opening provided on the heat-insulating main body 101 so as to be openable and closable, and a cooling unit which directly cools air inside the heat-insulating main body 101, and includes: a support 201 which is placed in the storage compartment and on which a photocatalyst is supported; the irradiation unit 202 which irradiates the support 201 with excitation light which excites the photocatalyst; an air blowing unit 203 which forcibly blows the air inside the storage compartment toward the support 201; and a flow path forming unit 124 which forms a flow path for the air blown by the air blowing unit 203, and the flow path forming unit has, in an intermediate part of the flow path for the air, a narrow portion which forms the throat which has a cross-sectional area narrower than in other portions, and the support 201 is placed in the narrow portion.

With this, the air forced through the flow path by the air blowing unit 203 is narrowed by the narrow portion. Accordingly, the air efficiently contacts the support 201 provided in the narrow portion, thus allowing increased sterilization and deodorization effects.

Furthermore, it is preferable that the air blowing unit 203 have an axial flow fan, which is placed such that a rotational axis of the axial flow fan intersects with a direction in which the cool air passes through the flow path.

With this, when the flow path is provided along the wall surface in the storage compartment, it is possible to reduce the amount of projection of the air blowing unit into the storage compartment as much as possible, and to thereby create sterilization and deodorization effects without largely scarifying the capacity of the storage compartment.

Furthermore, an absorption filter 204, which absorbs and holds an odor in the storage compartment, is included, and the absorption filter is placed downstream from the support 201 in the flow path such that a direction in which the cool air passes through the absorption filter intersects with the direction in which the cool air passes through the flow path.

With this, it is possible to improve the deodorization effect and so on, and furthermore, by simply providing the absorption filter downstream from the support 201, it becomes only necessary to hold the remaining odors after the photocatalytic process, thereby extending the life of the absorption filter. Furthermore, since the absorption filter is provided in a slanted state with respect to the flow path, it is possible to reduce as much projection as possible of the absorption filter into the storage compartment.

Thus, the refrigerator according to the present invention forcibly blows the air inside the storage compartment, sterilizes bacteria and so on contained in the air, and blows the sterilized air into the storage compartment. Accordingly, it is possible to sterilize the inside of the storage compartment as a whole by forcing the convection of the air inside the storage compartment. In addition to this, it is possible to perform sterilization and deodorization efficiently.

A refrigerator according to the present invention includes a rectangular heat-insulating main body 101 which is made of a heat-insulating material and has an opening in the front, and in which a storage compartment is formed, a door attached to the opening so as to be openable and closable, and a cooler 110 that is a cooling unit which directly cools air inside the heat-insulating main body 101, and the refrigerator includes: a support 201 which is placed in the storage compartment and on which a photocatalyst is supported; an irradiation unit which irradiates the support 201 with excitation light which excites the photocatalyst; and an air blowing unit 203 which forcibly blows the air inside the storage compartment toward the support 201, and the air blowing unit 203, the support 201, and the cooling unit are placed in order, starting from upstream of a flow of the air generated by the air blowing unit 203.

With this, the air having a relatively high temperature is allowed to contact the support 201, thus preventing generation of dew condensation on the support 201. Accordingly, it is possible to suppress the deterioration of the photocatalytic function, thereby making it possible to perform efficient sterilization and deodorization.

In addition, this placement, when adopted, does not disturb the natural convection generated by the cooling unit and thus allows formation of an air flow in a direction that promotes the natural convection, thereby making it possible to remove temperature irregularity within the storage compartment with high efficiency.

Furthermore, it is preferable that an absorption filter 204 which absorbs an odor in the storage compartment be included and that the absorption filter 204 be placed downstream from the support 201 and upstream from the cooling unit 110.

With this, it is possible to improve the deodorization effect and so on, and furthermore, by simply providing the absorption filter 204 downstream from the support 201, it is only necessary to hold the remaining odors after the photocatalytic process, thereby extending the life of the absorption filter 204.

As described above, in the refrigerator according to the present invention, the air having a relatively high temperature is sent toward a lower-temperature side. With this, it is possible to reinforce the natural convection inside the storage compartment. In addition, the photocatalyst is reacted to the air having a relatively high temperature, thus making it possible to perform efficient sterilization and deodorization.

### (Second Embodiment)

Next, another embodiment will be described. Note that in some cases, a structure common to the first embodiment described above will be appended with the same numeral, and the description thereof will be omitted.

FIG. 15 is a longitudinal sectional view schematically showing a refrigerator in the present embodiment.

As the figure shows, a sterilization apparatus 200 and a lighting unit 300 are attached to a ceiling of a heat-insulating main body 101. Note that the sterilization apparatus 200 and the lighting unit 300 are described later.

FIG. 16 is a cross sectional view of a sterilization apparatus and a lighting unit mounted to the refrigerator.

FIG. 17 is a bottom view of a sterilization apparatus and a lighting unit mounted to the refrigerator.

The lighting unit 300 is a unit which illuminates the inside of the heat-insulating main body 101 and includes: a light source 301, a light board 302, and a circuit board (not shown). The lighting unit 300 is provided closer to the heat-insulating door 107 than to the sterilization apparatus 200 in a concave portion 120 where the sterilization apparatus 200 is provided. The lighting unit 300 is provided in the heat-insulating main body 101 such that it can illuminate the inside of the heat-insulating main body 101, from the side of the heat-insulating door 107 toward a far corner of the heat-insulating main body 101.

The light source 301 is a light source for illuminating the inside of the heat-insulating main body 101, and a white LED is used as the light source 301 in the present embodiment.

The light board 302 is a printed board which holds a line of plural light sources 301 and on which wiring for supplying power to the light source 301 is printed. The light board 302 is held by the concave portion 120 by being engaged with an engagement stopper 303 implanted in the concave portion 120.

The circuit board includes a circuit for converting the power supplied to the refrigerator 100 into power appropriate for the light source 301. The circuit board also supplies power to a light source 207 which irradiates the support 201 with an excitation ray. In addition, the circuit board includes a circuit which detests opening or closing of the heat-insulating door 107 and supplies power to the light source 301 only when the heat-insulating door 107 is open.

FIG. 18 is a base view showing the sterilization apparatus and the lighting unit, which are covered with a cover.

The cover 206 is a plate-shaped member which can cover the whole sterilization apparatus 200 attached to the heat-insulating main body 101, and includes an inlet 251, an outlet 252, and a transparent window 213. In addition, louvers for controlling the air flow are attached to the inlet 251 and the outlet 252. The cover 206 improves design versatility by covering over the sterilization apparatus 200 and the lighting unit 300, and also insulates the sterilization apparatus 200 and the lighting unit 300 from the cool air inside the heat-insulating main body 101 by being placed at a predetermined distance from the sterilization apparatus 200 and the lighting unit 300. Accordingly, the cover 206 protects the sterilization apparatus 200 and the lighting unit 300 from dew condensation, thus preventing performance degradation of the sterilization apparatus 200. In addition, since the cover 206 also covers the lighting unit 300 embedded in the heat-insulating main body 101, the heat generated from the lighting unit 300 is introduced into the sterilization apparatus 200 rather than into the heat-insulating main body 101. Accordingly, the temperature of the air introduced into the sterilization apparatus 200 rises slightly higher than the temperature of the air inside the heat-insulating main body 101, so that sterilization and deodorization is promoted.

The transparent window 213 is a part provided opposite to the lighting unit 300 on the cover 206 and is transparent.

The portions other than the transparent window 213 of the cover 206 are semi-transparent like frosted glass, and the entire flow path forming unit 124 is also semi-transparent like frosted glass. This allows part of the visible right emitted from the light source 207 and the reflected light of the visible light reflected within the sterilization apparatus 200 to leak into the heat-insulating main body 101. With this, it is possible to check that the sterilization is actually performed, and to recognize from outside whether or not the irradiation unit 202 is in trouble.

The operation and effects of the refrigerator 100 thus configured will be described below.

The air containing odor components (such as an organic substance) or bacteria, which are present in the inside of the heat-insulating main body 101, passes through the inlet 251 of the cover 206 by the blowing force of the air blowing unit 203, and is introduced into the sterilization apparatus 200 via the air blowing unit 203. The introduced air passes licking a face of the support 201, which has a largest area. Here, more air contacts the support 201 because a flow path, which is sandwiched between the irradiation unit 202 and the flow path forming unit 124 that is placed opposite to the irradiation unit 202, is set narrower than the other portions. The odor components and bacteria contained in the air are captured on the surface of the support 201. The captured odor components and bacteria are deodorized and sterilized by oxidative decomposition caused by ordinary catalytic action (not by photocatalytic action) and the sterilization effect of the silver oxide. With this, since odor-decomposition and sterilization effects are produced by the silver-oxide action even when not irradiated with light, it is possible to reduce the volume of irradiance and the time for the irradiation while, at the same time, maintaining the desired deodorization and sterilization effects, thus achieving longer life and increased energy-saving effects of the irradiation unit. Furthermore, with the light energy (blue light or an ultraviolet ray) irradiated from the light source 207, the silver oxide having an absorption spectrum in these wavelength ranges is excited by the blue light energy, so that the photocatalyst on the surface of the support 201 is excited. When the photocatalyst is excited, hydroxyl (OH) radical is generated from moisture in the air, so that oxidative decomposition is performed on the odor components captured on the support 201 or present near the support 201 and the bacteria are lysed.

Furthermore, odor components which are not fully decomposed on the surface of the support 201 pass along with air through the pores of the absorption filter 204 having a honeycomb structure, and are absorbed and held by the absorption filter 204.

As described above, the air passing though the sterilization apparatus 200 becomes clean air that is deodorized and sterilized, and is blown into the heat-insulating main body 101 from the outlet 252 on the cover 206. Accordingly, by continuing the sterilization, it is possible to decrease the number of bacteria and reduce odor components.

On the other hand, inside the heat-insulating main body 101, overall air convection is promoted by introducing air into the sterilization apparatus 200 and deriving air from the sterilization apparatus 200. Accordingly, it is possible to prevent the cool air from staying at the bottom of the heat-insulating main body 101, thus making it possible to even out the temperature inside the heat-insulating main body 101. Furthermore, the OH radical generated by the sterilization apparatus 200 convectively flows along with the air in the heat-insulating main body 101, and performs deodorization and sterilization also in the heat-insulating main body 101.

As described above, the refrigerator can physically perform sterilization and deodorization using the sterilization apparatus 200, without using an organic sterilization agent or the like. Thus, it is no longer necessary to consider the influences of organic sterilization agents over a human body and so on. In addition, since the photocatalyst can perform sterilization and deodorization without changing itself, it is possible to maintain its effects semipermanently.

In addition, the lighting unit 300 lights up by detecting that the heat-insulating door 107 is opened. When the lighting unit 300 is on, the far corner of the heat-insulating main body 101 is illuminated from the side of the heat-insulating door 107, and storage items stored in the heat-insulating main body 101 are lit up from the front, thus making it very easy to see the storage items.

Note that in the embodiment described above, the irradiation unit 202 and the lighting unit 300 are directly attached to the heat-insulating main body 101, and the flow path is formed with the heat-insulating main body 101 and the flow path forming unit 124. However, the present invention is not limited to this. For example, as FIG. 19 shows, the sterilization apparatus may also be a unit-type lighting and sterilization apparatus which is attachable to and detachable from the heat-insulating main body 101 and in which the irradiation unit 202, the support 201, the air blowing unit 203, and the lighting unit 300 are attached to a box-shaped case. This makes it easier to attach or detach the lighting and sterilization apparatus to or from the refrigerator.

A refrigerator according to the present invention includes a heat-insulating main body 101 which is made of a heat-insulating material and in which a storage compartment is formed, a door attached to an opening provided on the heat-insulating main body 101 so as to be openable and closable, and a cooler 110 that is a cooling unit which directly cools air inside the heat-insulating main body 101, and includes a support 201 which is placed in the storage compartment and on which a photocatalyst is supported; an irradiation unit 202 which irradiates the support 201 with excitation light which excites the photocatalyst; an air blowing unit 203 which forcibly blows the air inside the storage compartment toward the support 201; a sterilization apparatus 200 including a flow path forming unit 124 which forms a flow path for the air blown by the air blowing unit 203; a cover 206 attached to an inner wall of the heat-insulating main body 101 and covering the sterilization apparatus 200; and a lighting unit 300 which is covered with the cover and illuminates an inside of the heat-insulating main body 101.

With this, the sterilization apparatus 200 and the lighting unit 300 are covered with the same cover 206, thus allowing sterilization, deodorization, and lighting of the storage compartment in a compact state. In addition, it is possible to efficiently use the heat generated by the lighting unit 300 for the sterilization and deodorization. In addition, the sterilization apparatus 200 and the lighting unit 300 can also share an electrical system and so on, thus achieving space saving and cost reduction.

In addition, it is preferable that the sterilization apparatus 200 be attached to a ceiling surface of the heat-insulating main body 101, and that the lighting unit 300 be attached closer to the door than to the sterilization apparatus 200.

In addition, it is preferable that the irradiation unit 202 have directivity toward an irradiation direction of light and be attached to the heat-insulating main body 101 such that the light is directed from the door side to an innermost side of the heat-insulating main body 101.

With these, when opening the door, the food and so on inside the storage compartment are lit up from the front, so that the food and so on is clearly recognized.

In addition, it is preferable that the lighting unit 300 be attached to the concave portion provided in the heat-insulating main body 101.

With this, it is possible to illuminate the inside of the storage compartment without largely scarifying the capacity of the storage compartment.

As described above, the refrigerator according to the present invention forcibly blows the air inside the storage compartment, sterilizes bacteria and so on contained in the air, and blows the sterilized air into the storage compartment. Accordingly, it is possible to sterilize the whole storage compartment by forcing convection of the air inside the storage compartment. Furthermore, since the inside of the storage compartment is illuminated with the lighting unit covered with the cover along with the sterilization apparatus, it is possible to provide a compact sterilization and lighting unit.

Hereinafter, another embodiment of the present invention will be described with reference to the drawings; however, in some cases, the same structure as described earlier in the preceding embodiments will be appended with the same numeral and the descriptions thereof will be omitted. Note that the present invention is not limited by this embodiment.

### (Third Embodiment)

FIG. 20 is a longitudinal sectional view of a refrigerator in the present invention.

FIG. 21 is an elevation view showing a structure of a flow path forming unit of the refrigerator in the present invention.

In these figures, a heat-insulating main body 101, which is insulated from the periphery by a heat-insulating material, for example, rigid foamed urethane, is divided into plural sections, and these plural sections are arranged in parallel as: a refrigerating compartment 102 provided in an uppermost part; a freezing compartment 103 provided in an lowermost part as a first storage compartment; a vegetable compartment 104 provided directly above the freezing compartment 103 as a second storage compartment; an ice compartment 105 provided between the vegetable compartment 104 and the refrigerating compartment 102 as a third storage compartment; and a switch compartment 106 provided as a fourth storage compartment. All of the freezing compartment 103, the vegetable compartment 104, the ice compartment 105, and the switch compartment 106 have a shape of a drawer-type storage compartment. A front opening on each of the storage compartments includes a heat-insulating door 107.

The vegetable compartment 104 is set to a temperature of 2 °C to 7 °C, which is equal or a little higher than the temperature in the refrigerating compartment 102. The lower the temperature, the longer the freshness of leafy vegetables can be preserved.

The ice compartment 105 is a storage compartment for storing ice, which is made by an ice machine (not shown) provided inside the ice compartment 105. The switch compartment 106 can switch between refrigerant zones from chilling to freezing temperatures according to the purpose, using a control panel attached to the main body of the refrigerator.

The top surface of the heat-insulating main body 101, which is provided with a concavity stepped toward a back face of the refrigerator, is made up of a first top surface 111 and a second top surface 112, and the concavity 113 which is stepped houses a compressor 114 and high-voltage components of a refrigeration cycle such as a dryer for removing water (not shown). In other words, the concavity 113 including the compressor 114 is formed cutting into a rear area of the uppermost part of the refrigerating compartment 102. Thus, the compressor 114 is not placed in a rear area of a lowermost part of the storage compartment of the heat-insulating main body 101 as is commonly seen conventionally.

A cooling room 115 is provided astride the back surfaces of the freezing compartment 103 and the vegetable compartment 104, and the cooling room 115 is separated from the freezing compartment 103 and the vegetable compartment 104 by a first partition 116 having heat-insulating properties as a heat-insulating wall. In addition, the freezing compartment 103 and the vegetable compartment 104 are separated by a second partition 117 having heat-insulating properties as a heat-insulating wall. Since the first partition 116 and the second partition 117 are components assembled after the foam formation of the heat-insulating main body 101, foamed polystyrene is normally used as a heat-insulating material. However, in order to improve heat-insulating performance and stiffness, rigid foamed urethane may also be used. Furthermore, a vacuum insulation material having high heat-insulation properties may be inserted to make the partition structure thinner. In addition, a third partition 118 that is the top surface of the ice compartment 105 and the switch compartment 106 arranged in parallel, and a fourth partition 119 provided at the bottom are integrally molded from the same foam insulation material as the material for heat-insulating main body 101.

Inside the cooling room 115, a typical fin-and-tube type cooler 110 is provided vertically long in a longitudinal direction, astride the freezing compartment 103 and the vegetable compartment 104 including a rear area of the second partition 117 that is the heat-insulating partition such that the placement ratio of the freezing compartment 103 is higher than the placement ratio of the vegetable compartment 104. A cooling fan 121 which blows cool air cooled by the cooler 110 into each compartment by a forced-convection method is placed in a space in an upper part of the cooler 110. The cool air cooled by the cooler 110 is carried into each compartment to be heat-exchanged therein, after passing through the flow path forming unit 124 for blowing the cool air to each compartment, and is returned to the cooler 110 through the flow path forming unit.

In FIG. 21, the temperatures in the refrigerating compartment 102, the ice compartment 105, and the switch compartment 106 are controlled using a dumper which intermittently controls the discharged cool air. Each of the refrigerating compartment 102, the ice compartment 105, and the switch compartment 106 is equipped with a temperature sensor (not shown) for controlling interior temperature: the control panel 122 opens the dumper when the temperature sensor indicates a temperature higher than predetermined high and low temperatures and closes the dumber when the temperature is lower, so as to adjust the interior temperature to a predetermined temperature.

The flow path forming unit has a structure, in which: an ice-compartment dumber 123 which intermittently controls the ice compartment 105 is provided in an upper part of the cooling room 115, and the cool air sent from the cooling fan 121 is blown into the ice compartment 105 after passing through the ice-compartment dumper 123 and the flow path forming unit 124 for blowing air into the ice compartment, and returns to the cooler 110 through the flow path forming unit 124 for returning to the ice compartment after undergoing heat exchange.

The dumper which intermittently controls the refrigerating compartment 102 and the dumper which intermittently controls the switch compartment 106 are assumed as a twin dumber 128 integrally structured with a flap 125 for the refrigerating compartment, which intermittently lets the cool air flow into the refrigerating compartment 102, a flap 125 for the switch compartment, which intermittently lets the cool air flow into the switch compartment 106, and a drive unit 127 which drives the flaps. The twin dumper 128 is placed in a back portion of the ice compartment 105 and the switch compartment 106. In addition, in the back surface of the refrigerating compartment 102, the flow path forming unit 124 including two outlets 252 is provided. In addition, in the flow path forming unit 124, a support 201 is placed near the outlet 252, and an irradiation unit 202 is placed opposite to the support 201. The support 201 is a member containing titanium oxide and functioning as a filter. In addition, the irradiation unit 202 is a UV-LED which emits an ultraviolet ray of 380 nm.

The operation and effects of the refrigerator thus configured will be described below.

First, the operation of the refrigeration cycle will be described. According to the set temperature inside the refrigerator, the refrigeration cycle is activated by a signal sent from the control board 122, so that cooling operation is performed. A refrigerant having high temperature and high pressure, which is discharged by the operation of the compressor 114, dissipates heat and is condensed into liquid in the condenser (not shown), and then reaches a capillary tube (not shown). Subsequently, in the capillary tube, the refrigerant is decompressed through heat exchange with a suction tube (not shown) leading into the compressor 114, and reaches the cooler 110 as a liquid refrigerant having low temperature and low pressure. By the operation of the cooling fan 121, the refrigerant inside the cooler 110 is heat-exchanged with the air inside the storage compartment and then evaporates, and the supply of the cool air having low temperature is controlled using the dumper and so on, so as to perform desired cooling of each compartment. The refrigerant blown out of the cooler 110 is sucked into the compressor 114 through the suction tube.

Next, the deodorization and sterilization effects will be described.

The cool air containing odors and bacteria, which is blown by the cooling fan 121, passes by the support 201 through the flap 125 for the refrigerating compartment and the flow path forming unit 124, and is blown into the refrigerating compartment 102 through the outlet 252. At this time, the odor components and bacteria contained in the cool air are captured on the support 201. Then, the titanium oxide on the surface of the support 201 is excited by the light energy of the ultraviolet ray emitted from the irradiation unit 202 and generates OH radical from moisture in the air, so that oxidative decomposition and lysis are performed, respectively, on the odor components and bacteria captured on the support 201. Thus, the cool air blown out of the outlet 252 is now clean air deodorized and sterilized, and is blown into the refrigerator. On the other hand, after undergoing heat exchange, the air returns to the cooler 110 via the flow path forming unit 124. In addition, in the structure, the cool air sent by the cooling fan 121 is blown into the switch compartment 106 after passing through the flap 125 for the switch compartment and the flow path forming unit 124 for blowing air into the switch compartment, and returns to the cooler 110 through the flow path forming unit 124 for returning to the switch compartment after undergoing heat exchange.

As described above, by placing the support 201 including a photocatalyst in an outlet portion of the flow path forming unit, and placing the irradiation unit 202 opposite to the support 201, the odor components and bacteria captured on the supported 201 are decomposed by photocatalytic action immediately before the cool air blows into the refrigerator, and thus, the refrigerator of the present embodiment can constantly keep cleanliness of the cool air blowing into the refrigerator.

### (Fourth Embodiment)

FIG. 22 is an elevation view of the refrigerator in the present invention.

FIG. 23 is a longitudinal sectional view of a flow path forming unit including a support in the present invention.

In these figures, the flow path forming unit 124 has a structure of a T-shaped air path, in which an outlet 252 is provided on a side in an upper part of the refrigerating compartment 102.

The support 201 having silver oxide is provided in such a manner as to contact a wall of the flow path forming unit at a point where a flow of cool air becomes refracted from a vertical direction that is a bottom-to-top direction with respect to the refrigerating compartment 102, into a horizontal direction with respect to the refrigerating compartment 102.

The irradiation unit 202 is a blue LED which emits light having a wavelength range of 470 nm, and is provided opposite to the support 201 in a concavity formed below the support 201 in the flow path forming unit.

According to the structure, the walls of the flow path forming unit 124 around the irradiation unit 202 are formed with a light-transmissive member 133, and both the support 201 and the inside of the refrigerating compartment 102 are irradiated with the light from the irradiation unit 202.

The operation and effects of the refrigerator thus configured will be described below.

The cool air containing odor components and bacteria flows up within the flow path forming unit 124, hitting the support 201 provided at the end of the flow path forming unit and changing itself into a whirling flow of the cool air, so that the cool air contacts the surface of the support 201 at different angles. As a result, the contact ratio with respect to the support 201 is increased, thus increasing the capturing ratio of odor components and bacteria contained in the cool air with the support 201. The odor components and bacteria captured in the support 201 are deodorized and sterilized by the oxidative decomposition by silver oxide and the antibacterial action of the support 201. Furthermore, when irradiated with the blue LED that is the irradiation unit 202, the silver oxide having an absorption spectrum within the wavelength range of blue light becomes excited by the blue light energy to become photocatalytically active, and powerfully performs deodorization and sterilization with strong oxidation reaction and bacteriolytic reaction of the generated OH radical.

In addition, since it is covered with a cover made of a light-transmissive member 133, the irradiation unit 202 (blue LED) irradiates both the surface of the support 201 and the inside of the refrigerating compartment 102.

As described above, in the refrigerator of the fourth embodiment, the support 201 is provided on a wall of a bent portion of the T-shaped flow path forming unit 124, so that the support 201 does not become resistance to the air path and also enhances the contact ratio with cool air, thus allowing high deodorization and sterilization performance.

In addition, in the refrigerator of the present invention, it is possible to perform sterilization using silver oxide as a photocatalyst and using blue light as the light source. This allows sterilization under such a low-temperature environment as in the refrigerator where microorganisms grow slowly, using a photocatalytic activity of the silver oxide having a slight absorption spectrum for the blue light, and a sufficient photocatalytic effect can be produced even with such a longer-life and inexpensive blue LED.

In addition, part of the wall surface of the flow path forming unit 124 is made of the light-transmissive member 133, the irradiation unit 202 (blue LED) is provided on an inner side of the refrigerating compartment 102, and the irradiation unit 202 is used both as a light for illuminating the inside of the refrigerating compartment 102 and as an excitation light source for exciting the photocatalyst on the support 201. With this configuration, it is possible to use one light source for both exciting the photocatalyst of the filter and illuminating the inside of the refrigerating compartment 102, thus saving the internal space of the refrigerator.

In addition, the present embodiment has described the case where the flow path forming unit 124 for blowing air into the refrigerating compartment is T-shaped, but the same effect can be produced even when the unit is L-shaped.

According to the invention as described above, the refrigerator includes a heat-insulating main body which includes a storage compartment, a flow path forming unit which forcibly makes the air flow, a support having a photocatalyst, and an irradiation unit which irradiates the photocatalyst, and the support is placed in an outlet portion in the flow path forming unit.

With this, odor components and bacteria captured on the support are decomposed by photocatalytic action immediately before the cool air blows into the refrigerator, thereby making it possible to constantly keep cleanliness of the cool air blowing into the refrigerator, and thus allowing provision of a refrigerator having higher usability and quality.

Furthermore, the irradiation unit is placed opposite to the support at a predetermined distance.

With this, by separating the irradiation unit from the support at the predetermined distance, it is possible to increase an area of the support to be irradiated with the light from the irradiation unit, thus allowing a more extensive photocatalytic activity.

Furthermore, the outlet portion in the flow path forming unit has an elbow portion, and the support is placed in the elbow portion on the flow path forming unit.

With this, compared to a conventional case of placing the support perpendicular to the air flow direction, the flow of the cool air in the air path changes and then causes turbulence in the elbow portion with reduced pressure loss for the air path. Thus, as the cool air stays longer on the surface of the support provided in the elbow portion, the frequency of contact between the photocatalyst on the support and the cool air increases, so that the photocatalytic reaction is increased.

In addition, the support is placed on the wall surface of the flow path forming unit opposite to the air flow in the elbow portion.

With this, all the cool air within the air path hits the support to cause turbulence in the cool air in front of the support and cause the cool air to stay longer on the surface of the support, thus increasing the frequency of the contact between the photocatalyst on the support and the cool air, so that the photocatalytic action is increased.

Furthermore, it is assumed that the support is a deodorization support which deodorizes the air in the refrigerator.

With this, it is possible to deodorize the odor components contained in the cool air with the deodorization support, and to blow odorless cool air into the refrigerator.

Furthermore, it is assumed that the support is a support which sterilizes the air in the refrigerator.

With this, the bacteria contained in the cool air can be captured using the support and lysed with the OH radial generated by the activation of the photocatalyst, thus allowing clean air to blow into the refrigerator.

Furthermore, it is assumed that the photocatalyst is silver oxide.

With this, even when not irradiated with light, odor-decomposition and antibacterial effects are produced by the silver-oxide action, and when irradiated with light, OH radical generated by the photocatalytic reaction of the silver oxide further performs powerful deodorization and sterilization and also extends the life and energy-saving effect of the irradiation unit.

Furthermore, the irradiation unit includes a light source including light having a wavelength of an ultraviolet or blue-light wavelength range.

With this, the silver oxide, which has a slight absorption wavelength even in the blue-light range in addition to the ultraviolet range, becomes excited by the light of the blue-light wavelength range and can therefore produce a sufficient photocatalytic effect with such a longer-life, inexpensive blue LED under such a low-temperature environment as in the refrigerator where microorganisms grow slowly.

Furthermore, part of the wall surface of the flow path forming unit in the outlet portion is made of a light-transmissive member, and the irradiation unit is provided in the light-transmissive member on an inner side of the refrigerating compartment, and the irradiation unit is used both as a light for illuminating the inside of the refrigerating compartment and as an excitation light source for exciting the photocatalyst on the support.

With this, it is possible to use one light source for both exciting the photocatalyst of the support and illuminating the inside of the refrigerating compartment, thus saving the internal space of the refrigerator.

### (Fifth Embodiment)

Next, another embodiment of a refrigerator according to the present invention will be described with reference to the drawings.

FIG. 24 is an elevation view of a refrigerator in a fifth embodiment of the present invention.

As the figure shows, the refrigerator 100 according to the present embodiment is a refrigerator 100 having French doors, and includes a storage compartment separated into upper and lower parts in a heat-insulating main body 101.

The storage compartment included in the refrigerator 100 is separated into plural sections, and such plural sections are referred to as, according to the function (cooling temperature): a refrigerating compartment 102, an ice compartment 105, a switch compartment 106 which is included in the ice compartment 105 and allows change of interior temperature, a vegetable compartment 104, and a freezing compartment 103, and so on.

At the front opening of the refrigerating compartment 102, a rotational heat-insulating door 107 filled with foam insulation such as urethane is provided.

In addition, the ice compartment 105, the switch compartment 106, the vegetable compartment 104, and the freezing compartment 103 each include a rotational heat-insulating door 107 that serves as a front panel of a drawer, with which the storage compartment is concealed to prevent leakage of cool air.

FIG. 25 is a longitudinal cross-sectional view of the refrigerator in the present embodiment, taken along from line A-A in FIG. 24.

A cooling fan 121 is provided in a space in an upper part of the cooler 110. The cooling fan 121 blows the cool air cooled by the cooler 110, forces a convective flow of the cool air in each storage compartment and circulates the cool air in the refrigerator 100.

Inside the refrigerator 100, a circulation path in which the cool air is forcibly circulated is formed. Specifically, the cool air cooled by the cooler 110 is forcibly blown by the cooling fan 121 to be carried into each compartment through a flow path forming unit provided between each compartment and the heat-insulating main body 101; then, the cool air cools each compartment and is returned to the cooler 110 through the flow path forming unit.

Note that the cool air is circulated using one cooling fan 121.

FIG. 26 is a diagram showing the structure of the flow path forming unit, which is included in the circulation path of the cool air in the present embodiment.

As the figure shows, the refrigerator 100 includes: a circulation path for the refrigerating and vegetable compartments in which cool air having a relatively high temperature flows; and an ice compartment circulation path, a freezing compartment circulation path, and a switch compartment circulation path in which relatively low-temperature cool air flows.

First, the circulation path for the refrigerating and vegetable compartments will be described.

The air cooled by the cooler 110 is sent by the cooling fan 121 to the refrigerating compartment 102 through a flow path forming unit 124.

However, the cool air cooled by the cooler 110 is cooled down to a temperature that is sufficiently low for the freezing compartment 103. Therefore, when the air having a relatively low temperature is continuously sent to the refrigerating compartment 102, the refrigerating compartment 102 is cooled down too much. So, a twin dumber 128 which can control cool-air insertion is provided in the cool-air circulation path including the refrigerating compartment 102. The twin dumber 128 controls the insertion of the cool air (ON and OFF of the cool air flow) cooled by the cooler 110, and the cool air does not constantly flow in the path for refrigerating compartment 102 and the vegetable compartment 104. In addition, when the whole refrigerator 100 is sufficiently cooled down, the cooling fan 121 stops rotating, and the circulation of the cool air stops accordingly. At this time, a refrigeration cycle, that is, a compressor 114 or the like also stops.

The cool air cooled by the cooler 110 flows, from bottom to top, through the flow path forming unit 124, and is blown into the refrigerating compartment 102 through the outlet 242 which opens in an upper part of the refrigerating compartment 102. The air, having passed through the refrigerating compartment 102, is sucked into the collection opening 131 which opens in a lower part of the refrigerating compartment 102. Next, the cool air sucked into the collection opening 131 is discharged into the vegetable compartment 104. Lastly, the cool air, passing through the vegetable compartment 104, returns to the cooler 110. Thus, the circulation path for the refrigerating compartment 102 and the vegetable compartment 104 has been described.

Note that in the present embodiment, other than the path of the cool air blown into the freezing compartment 102 through the outlet 252, part of the cool air is introduced into the sterilization apparatus 200 and branched before it is blown out from the outlet 252 into the freezing compartment 102, and then returns to the circulation path for the freezing compartment 102 and the vegetable compartment 104. The branch path making up part of the circulation path of the cool air will be described later.

In addition, the cool-air circulation in the ice compartment 105 and the switch compartment 106 is also controlled by the dumper which intermittently controls the cool air that is blown out, so that temperature in each compartment is controlled. Note that each of the refrigerating compartment 102, the ice compartment 105, and the switch compartment 106 is equipped with a temperature sensor (not shown) which controls the interior temperature, and the opening and closing of the dumper is controlled by the control board 122 (see FIG. 25) attached in a back portion of the refrigerator 100. In other words, the dumber is caused to open when the temperature sensor indicates a temperature higher than a predetermined first temperature, and to be closed to adjust the interior temperature to a predetermined temperature when the indicated temperature is lower than a second temperature.

The flow path forming unit has a structure in which: an ice-compartment dumber 123 which intermittently controls the ice compartment 105 is provided in an upper part of the cooling room 115; and the cool air sent from the cooling fan 121 passes through the ice-compartment dumper 123 and the flow path forming unit 124 for blowing air into the ice-compartment and is blown into the ice compartment 105, and returns, after undergoing heat exchange, to the cooler 110 through the flow path forming unit 124 for returning to the ice compartment.

The twin dumber 128 integrally includes a dumber which intermittently controls the refrigerating compartment 102 and a dumper which intermittently controls the switch compartment 106, and further includes a flap 125 for the refrigerating compartment which intermittently lets the cool air flow into the refrigerating compartment 102, a flap 125 for the switch compartment 106 which intermittently lets the cool air flow into the switch compartment 106, and additionally integrally includes a drive unit 127 which drives the flaps. The twin dumber 128 is provided around the back surface of the ice compartment 105 and the switch compartment 106.

Next, the sterilization apparatus 200 will be described.

FIG. 27 is a perspective view showing a sterilization apparatus mounted to the refrigerator in the present embodiment.

The sterilization apparatus 200 of the present embodiment is an apparatus which can forcibly sterilize the bacteria and spores that are present in the cool air and can also achieve deodorization by decomposing an organic substance present in the cool air. The sterilization apparatus 200 includes: a support 201 which is a support on which a photocatalyst is supported and an irradiation unit 202 which irradiates the support 201 with an excitation ray which excites the photocatalyst.

The support 201 is a porous resin member which allows increased contact with the air, and also is a filter member made of tangling fibers into which the photocatalyst is kneaded. In addition, for a base-material resin, a resin transmitting light having a wavelength which easily excites the photocatalyst is used.

The photocatalyst is a catalyst which, by irradiation of light having a given wavelength, can sterilize the bacteria in the cool air and deodorize odor components (such as an organic substance) in the cool air by oxidation, decomposition, or the like. The photocatalyst is considered as a substance which activates the component in the cool air (for example, ionization or radicalization) and can perform sterilization or deodorization based on such activation. Specifically, silver oxide or titanium oxide can be given as an example of the photocatalyst.

The light required for the silver oxide to fulfill its sterilization and other functions has a visible blue-color wavelength range approximately 400 nm to 580 nm. In addition, the light required for the titanium oxide to fulfill its sterilization and other functions has a wavelength 380 nm.

The irradiation unit 202 is an apparatus equipped with a light source 207 capable of emitting light having a wavelength which can excite the photocatalyst.

It is sufficient that the light source 207 can emit a predetermined amount of light having a wavelength, which includes light having the wavelength described above, and an ultraviolet lamp or an ordinary electric bulb can be given as an example. In addition, when the photocatalyst is silver oxide, it is possible to achieve longer life and cost reduction by adopting an LED which emits light in blue (470 nm) in a visible light range. In addition, when the photocatalyst is silver titanium, it is also possible to adopt a UV-LED which emits an ultraviolet ray of 380 nm.

In the present embodiment, silver oxide is used as the photocatalyst, and three LEDs lined up on a strip of board are used as the light source 207 for the irradiation unit 202.

Next, an attachment mode of the sterilization 200 to the refrigerator 100 will be specifically described.

FIG. 28(a) and (b) are cross-sectional views showing the attachment mode of the sterilization apparatus in the present embodiment.

As these figures show, a housing space 223 having an opening in a front face is provided in an upper end of the refrigerating compartment 102 and between two outlets 252. The housing space 223 is provided in a frontal part of a stepped concavity 113 to which the compressor 114 and so on are attached. The portion is an invalid space which does not serve as a storage space; therefore, by providing the housing space 223 in which to attach the sterilization apparatus 200, it is possible to attach the sterilization apparatus 200 without sacrificing the storage space in the refrigerating compartment 102.

On both of the walls of the housing space 223, a branch opening 255 for branching cool air is provided. The branch opening 255 introduces part of the cool air blown into refrigerating compartment 102, into the sterilization apparatus 200 as indicated by arrows in FIG. 28.

The irradiation unit 202 is embedded such that the light source 207 (LEDs) is horizontally lined up on the back wall of the housing space 223 so that the light of the light source 207 can be emitted toward the support 201 in front. Note that a floodlighting direction of the light source 207 has directional characteristics, and also spreads at a predetermined at a solid angle. Accordingly, the distance between the light source 207 and the support 201 is determined to a predetermined distance.

The frontal opening part of the housing space 223 is covered with a cover 206 having heat-insulating properties. The cover 206 is a plate-shaped member which curves bulging into the direction of the refrigerating compartment 102. In addition, as FIG. 28 shows, the cover 206 is more projected in the center of the refrigerating compartment 102 than in the frontal part of the back wall (the dotted and dashed line in the figure). This curve in the cover 206 creates a gap between the frontal part of the back wall of the refrigerating compartment 102 and the cover 206. The gap forms a through hole 257 which vertically connects the interior portion of the sterilization apparatus 200 and the refrigerating compartment 102.

With this through hole 257, it is assumed that the cool air introduced into the housing space 223 is blown out of the through hole 257. In addition, when the cooling fan 121 is stopped and the cool air is not forcibly circulated, it is assumed that the natural convection of the cool air causes the cool air inside the refrigerating compartment 102 to flow in and out of the sterilization apparatus 200 through the through hole 257.

An upper end edge 206a and a lower end edge 206a of the cover 206 are semi-transparent like frosted glass, and it is assumed that part of the light from the light source 207 or the reflected light that is reflected inside the sterilization apparatus 200 leaks out from the upper end edge 206a and the lower end edge 206a of the cover 206.

The cover 206 protects the support 201 and the irradiation unit 202, which are attached opposite to the refrigerating compartment 102, from dew condensation. In other words, dump air is introduced from the outside of the refrigerator 100 each time the heat-insulating door 107 is opened. Thus, without the cover 206, dew condensation is more likely to be generated when such dump air directly contacts the support 201 and the irradiation unit 202 of which temperatures are lower than the outer temperature. Thus, the cover 206 prevents the outer air from directly contacting the support 201 and the irradiation unit 202, so as to avoid dew condensation. In addition, such heat-insulating properties of the cover 206 also prevent the dew condensation on the cover 206 itself.

The support 201 is held on a tip of the supporting member 256, which is provided in a state of being protruded from the back surface of the cover 206 in a posterior direction. The support 201 is held such that a face having a largest area is a vertical face and such that the face having the largest area is placed opposite to the irradiation unit 202, so as to be efficiently irradiated with light from the light source 207.

With this, the support 201 is almost floating in the air, thus allowing almost all the area of the support 201 to be exposed to the air. This also prevents, as much as possible, the dew condensed on another member from moving onto the support as a result of having contact with the other member. Furthermore, it is not necessary to form a complex shape on the main body of the refrigerator 100 but only to form, in the cover 206, the supporting member 256 which is very small as compared to the refrigerating compartment, thus making it possible to curb the rise in production costs.

In addition, the support 201 has its front part supported by a large number of projections 208 provided on the back surface of the cover 206.

With this, since the support 201 neither flaps in the cool air nor bends itself due to gravity, it is possible to hold the surface of the support 201 upright and avoid generation of a shadow portion which is out of reach of the light from the irradiation unit 202, thus enabling an efficient excitation of the photocatalyst supported on the support 201.

In addition, since the face having the largest area of the support 201 is a vertical face, even when the dew is condensed on the support 201, the water condescend on the face having the largest area is pulled downward due to gravity, so that the face having the largest area is drained. Accordingly, it is possible to sterilize and deodorize the branched air by the photocatalyst, without being interrupted by the dew condensation water.

The operation and effects of the refrigerator 100 thus configured will be described below.

First, the operation of the refrigeration cycle will be described. According to the set temperature in each storage compartment, the refrigeration cycle is activated by a signal sent from the control board 122, so that cooling operation is performed. A refrigerant having high temperature and high pressure, which is discharged by the operation of the compressor 114, dissipates heat and is condensed into liquid in the condenser (not shown), and then reaches a capillary tube (not shown). Subsequently, in the capillary tube, the refrigerant is decompressed through heat exchange with a suction tube (not shown) leading into the compressor 114, and reaches the cooler 110 as a liquid refrigerant having low temperature and low pressure. By the operation of the cooling fan 121, the refrigerant inside the cooler 110 is heat-exchanged with the air inside the storage compartment and then evaporates, and the supply of the cool air having low temperature is controlled using the dumper and so on, so as to perform desired cooling of each compartment. The refrigerant blown out of the cooler 110 is sucked into the compressor 114 through the suction tube.

Note that as FIG. 28(b) shows, the flow and pressure of the cool air may be changed using a narrow portion, which is formed by forming a bulge in a face opposite to the support 201. Wit this, more cool air contacts the support 201, thus improving the sterilization and deodorization effects.

Next, the effects of the function of the sterilization apparatus 200 will be described.

The odor (organic substance and so on) sent from the cooling fan 121 is blown into the storage compartment 102 from the outlet 252, after passing through the flap 125 for the refrigerating compartment and the flow path forming unit 124 for blowing out the cool air. At this time, part of the cool air is branched to be introduced into the sterilization apparatus 200. The introduced air passes licking a face having a largest area of the support 201. Odor components and bacteria contained in the air are captured on the surface of the support 201. The captured odor components and bacteria are deodorized and sterilized by the oxidative decomposition and the sterilization effect produced by the silver oxide. With this, since the odor decomposition and the sterilization effect are produced by the silver-oxide action even when not irradiated with light, it is possible to reduce the volume of irradiance and the amount of time for the irradiation while, at the same time, maintaining the desired deodorization and sterilization effects, thus achieving extended life and increased energy-saving effects of the irradiation unit. Furthermore, with the light energy (blue light or an ultraviolet ray) irradiated from an LED (light source) 132, the silver oxide having an absorption spectrum in these wavelength ranges is excited by the blue light energy, so that the photocatalyst on the surface of the support 201 is excited. When the photocatalyst is excited, hydroxyl (OH) radical is generated from moisture in the air, so that the odor components captured on the support 201 are decomposed by oxidative decomposition and the bacteria are lysed.

As described above, the cool air passing through the sterilization apparatus 200 is blown into the refrigerator through the through hole 257 as clean air which is deodorized and sterilized. Then, inside the refrigerating compartment 102, the cool air is mixed with the cool air blown out of the outlet 252 and circulates in the circulation path.

In addition, the OH radical generated by the sterilization apparatus 200 is also blown into the refrigerating compartment 102 and so on along with the cool air, and performs deodorization and sterilization also in the refrigerating compartment 102.

Next, another mode of the sterilization apparatus 200 will be described.

FIG. 29 is a perspective view showing another attachment mode for the sterilization apparatus in the present embodiment.

As the figure shows, the sterilization apparatus 200 further includes: a light source cover 212 as a second cover, and a double cover 211 as a third cover. Note that the same members and so on as in the sterilization apparatus 200 described above will be appended with the same numerals, and the descriptions thereof will be omitted.

The light source cover 212 is a semi-cylindrical member, which covers the irradiation unit 202 in a sealed state in which cool air is not flowing, and is provided for protecting the irradiation unit 202 from dew condensation. Any material which can transmit the light which excites the photocatalyst may be used for the light source cover 212.

With this light source cover 212, the irradiation unit 202 is completely covered, thus making it possible to completely prevent the irradiation unit 202 from dew condensation. Particularly, this can protect the light source 207 including an electrical system.

The double cover 211 is used for making the cover covering the sterilization 200 into a trilaminar structure including an air layer.

This double cover 211 renders the cover covering the sterilization apparatus 200 a double cover and forms the air layer, so that its air-insulating function can be fulfilled. Accordingly, it is no longer necessary to use a high-insulating material for forming the cover 206 and the double cover 211, and thus it is possible to increase flexibility in selecting the material for the cover 206 and the double cover 211. For example, it is possible to form the cover 206 or the double cover 211 with a transparent material, and to check, through the entire cover from outside the sterilization apparatus 200, whether or not the irradiation unit 202 is emitting light.

As described above, when opening the heat-insulating door 107 of the refrigerator 100, the blue light from the back of the refrigerating compartment 102 can be seen, thus making it possible to check the operation status of the sterilization apparatus 200 without providing a separate display apparatus. In addition, it is possible to recognize that the refrigerator 100 is being sterilized with the light, thus making it possible to provide a sense of reassurance and a sense of cleanliness to a user of the refrigerator 100.

Furthermore, in the case of silver oxide, a long-life blue LED can be used as a light source for excitation, thus making it possible to secure stable performance for a long time and also to contribute to cost reduction.

As described above, according to the present invention, the circulating cool air is deodorized and sterilized by providing, in a circulation path of the cool air, the sterilization apparatus including a support 201 on which a photocatalyst is supported and an irradiation unit 202 which irradiates the support 201 using excitation light for exciting the photocatalyst. Thus, it is possible to produce the deodorization and sterilization effects extensively in the refrigerator, thus making it possible to keep the inside of the refrigerator in a sterilized status with least odor.

In addition, it is preferable that the support 201 be plate-shaped, and that the support 201 be placed such that a normal line 201a to a face having a larger area (see FIG. 27) intersects with the flow direction of the cool air.

With this, it is possible to perform sterilization in a condition which is not likely to increase the resistance to the air path of the circulating cool air. In addition, since the face of the support and the flow direction of the cool air are almost parallel to each other, and this allows the cool air to pass licking the support, it is possible to reliably increase an amount of contact between the support and the cool air.

In addition, compared to a conventional case where the filter is vertically provided blocking the flow path forming unit with respect to the air flow direction, it is possible to perform sterilization by suppressing pressure loss for the air path.

It is preferable that the support be plate-shaped and placed with a face having a larger area as a vertical face.

With this, even when dew condensation is generated on the surface of the support, the dew falls off the support due to gravity, thus making it possible to keep the surface of the support dewless extensively. Accordingly, it is possible to reduce performance decline as much as possible which is caused by the dew condensation on the sterilization apparatus.

It is preferable that the sterilization apparatus be provided inside the storage compartment and include a cover which covers the sterilization apparatus.

With this, even when dump, high-temperature air flows into the refrigerator when the refrigerator door is opened, it is possible to protect the sterilization apparatus from dew condensation.

In addition, it is preferable that the sterilization apparatus be attached inside the storage compartment and include a second cover which covers the irradiation unit.

With this, it is possible to effectively protect the irradiation unit which includes an electrical system, from dew condensation.

It is preferable that the support be placed between the irradiation unit and the cover, and that the cover include a support member provided in the cover in a protruded state and supporting the support, and a projection holding the cover and the support with a predetermined space.

With this, it is possible to hold the support floating state in the air and also prevent the deformation of the support due to the protrusion and so on. Accordingly, since almost the entire support contacts the cool air, it is possible to enhance the sterilization effect.

The sterilization apparatus may further include a third cover placed at a predetermined distance from the cover.

With this, it is possible to insulate the sterilization apparatus without using a heat-insulating material for the cover. Therefore, it is possible to increase flexibility in selecting the material for the cover, which allows, for example, use of a transparent cover.

The cover may include a through hole which connects the inside of the storage compartment and the inside of the sterilization apparatus.

With this, it is possible to flow the air in the sterilization apparatus by the natural convection generated in the refrigerator.

### (Sixth Embodiment)

Next, another embodiment of a sterilization apparatus 200 attached in another mode will be described.

FIG. 30 is a diagram showing a sterilization apparatus in an embodiment of the present invention with a back wall of a refrigerating compartment partially cut away.

As the figure shows, the sterilization apparatus 200 is provided in a flow path forming unit 124 that is a circulation path of cool air.

A support 201 in the present embodiment is shaped by inflecting, at a middle point in a longitudinal direction, a thin plate-shaped body of which a face having a largest area has a rectangle shape and which is V-shaped as seen from a lateral side (parallel to the face having the largest area). The support 201 is provided projecting toward an upstream of the flow direction of the cool air, and it is assumed that the support 201 separates and diverts the flow direction of the cool air into two directions. Note that in the present DESCRIPTION and Claims the term "inflecting" is used in a wider sense and includes curving.

Three light sources 207 included in an irradiation unit 202 are arranged in a position which allows efficient irradiation of the support 201 with light. Each of these light sources 207 is separately provided in the flow path forming unit 124 in such a manner as not to become an obstacle to the flow path of the cool air.

According to the sterilization apparatus 200 in the present embodiment, it is possible to increase the contact ratio between the support 201 and the cool air, thereby improving bacterial-killing ability and deodorization ability, and also to prevent, as much as possible, increase in resistance to the air path in the case where the sterilization apparatus is placed in the flow path forming unit 124.

Note that in the present embodiment, the case of branching the cool air by the support 201 is described, but the present invention is not limited to this: as the cross-sectional view in FIG. 31 shows, a support 201 having an inflected shape may be provided such that the air path is diverted by flowing the cold air along the concavity.

As described above, the support 201 may have an inflected shape and may be placed in such a manner as to divert the flow direction of the cool air.

By diverting the flow direction of the cool air with the support 201, it is possible to increase the amount of contact between the support 201 and the cool air without increasing the resistance to the air path, and thus increase the deodorization ratio and the sterilization ratio with respect to the odors and bacteria contained in the cool air.

Especially, the cool air flow in the air path is changed and then causes turbulence at a point where the cool air flow is diverted. Thus, it is possible to make the cool air stay longer on the surface of the support 201 by providing the support 201 at the point so as to divert the cool air more positively toward the support 201. Accordingly, the frequency of contact between the photocatalyst on the support 201 and the cool air is increased, so that the photocatalytic reaction is increased.

The refrigerator described above includes a heat-insulating main body which is made of a heat-insulating material and in which a storage compartment is formed, a door attached to an opening provided on the heat-insulating main body so as to be openable and closable, a cooling unit which cools air inside the heat-insulating main body to generate cool air, and a cool air circulation path in which the cool air circulates between the storage compartment and the cooling unit, and the refrigerator includes, in the cool air circulation path, a sterilization apparatus including a support on which a photocatalyst is supported and an irradiation unit which irradiates the support with excitation light which excites the photocatalyst.

With this, the circulating cool air is deodorized and sterilized, and thus it is possible to produce the deodorization and sterilization effects extensively in the refrigerator, thereby making it possible to maintain the inside of the refrigerator in a sterilized status with least odor.

In addition, this is unlikely to affect the resistance to the air path and can produce various effects, such as protecting the sterilization apparatus from dew condensation in addition to keeping the circulation efficiency of the cool air even when the sterilization apparatus is placed in the storage compartment.

In addition, it is preferable that the support 201 be plate-shaped and be placed such that a normal line to a face having a larger area intersects with the flow direction of the cool air.

With this, it is possible to perform sterilization in a condition that is unlikely to increase the resistance to the air path of the circulating cool air. In addition, this makes the face of the support and the flow direction of the cool air almost parallel to each other, thereby making it possible to secure an increased amount of contact between the support and the cool air.

In addition, compared to a conventional case where the filter is vertically provided blocking the flow path forming unit with respect to the air flow direction, it is possible to perform sterilization by suppressing the pressure loss for the air path.

In addition, the support may have an inflected shape and may be placed in such a manner as to divert the flow direction of the cool air.

By diverting the flow direction of the cool air with the support, it is possible to increase the amount of contact between the support and the cool air without increasing the resistance to the air path, and to thus increase the deodorization ratio and the sterilization ratio with respect to the odors and bacteria contained in the cool air.

Especially, the cool air flow in the air path is changed and then causes turbulence in an elbow portion which diverts the cool air flow. Thus, it is possible to make the cool air stay longer on the surface of the support 201 by providing the support at the point so as to divert the cool air more positively toward the support. Accordingly, the frequency of contact between the photocatalyst on the support and the cool air is increased, so that the photocatalytic reaction can be increased.

It is preferable that the support be plate-shaped and be placed with a face having a larger area as a vertical face.

With this, even when dew is condensed on the surface of the support, the dew falls off the support due to gravity, thus making it possible to keep the surface of the support dewless extensively. Accordingly, it is possible to reduce performance decline as much as possible which is caused by the dew condensation on the sterilization apparatus.

It is preferable that the sterilization apparatus be attached inside the storage compartment and include a cover which covers the sterilization apparatus.

With this, even when dump, high-temperature air flows into the refrigerator when the refrigerator door is opened, it is possible to protect the sterilization apparatus from dew condensation.

In addition, it is preferable that the sterilization apparatus be attached inside the storage compartment and include a cover which covers the sterilization apparatus.

With this, it is possible to effectively protect the irradiation unit which includes an electrical system from dew condensation.

It is preferable that, in the sterilization apparatus, the support be placed between the irradiation unit and the cover, and that the cover include a support member provided in the cover in a protruded state and supporting the support, and a projection holding the cover and the support with a predetermined space.

With this, it is possible to hold the support floating in the air and also prevent the deformation of the support due to the protrusion and so on. Accordingly, since almost the entire support contacts the cool air, it is possible to improve the sterilization effect.

The sterilization apparatus may further include a third cover provided at a predetermined distance from the cover.

With this, it is possible to insulate the sterilization apparatus without using a heat-insulating material for the cover. Therefore, it is possible to increase flexibility in selecting the material for the cover, which allows, for example, use of a transparent cover.

The cover may include a through hole which connects the inside of the storage compartment and the inside of the sterilization apparatus.

With this, it is possible to flow the air in the sterilization apparatus by the natural convection generated in the refrigerator.

### (Seventh Embodiment)

FIG. 32 is a control diagram according to the present invention.

As the figure shows, when a switch 254 (see FIG. 6), which is provided in a frontal part of a heat-insulating door 107 of a refrigerator 100, is turned ON to the operation side, both an air blowing unit 203 and an irradiation unit 202 start operating, so that the air which contains odors (such as an organic substance) and bacteria is introduced into a sterilization apparatus 200 by the air blowing unit 203.

In the sterilization apparatus 200, with the light energy (blue light or an ultraviolet ray) emitted from the irradiation unit 202 onto the surface of the support 201, the silver oxide having an absorption spectrum in these wavelength ranges is excited by the blue light energy, so that OH radial is generated from moisture in the air.

When the introduced air passes licking the surface of the support 201, the bacteria are captured by the support 201 and then lysed by this OH radical.

Furthermore, when the air passes through an absorption filter 204, chemical change of the odor components is induced to perform deodorization. In addition, the air, which is now clean, deodorized and sanitized air, is blown toward the cooler 110 that is cooled and undergoes heat exchange, so that the clean cool air circulates in a refrigerating compartment 102.

Note that the switch 254 is an apparatus provided in a frontal part of the heat-insulating door 107 of the refrigerator 100 and can start and stop operation of the air blowing unit 203 and the irradiation unit 202.

In addition, when the operation of the air blowing unit 203 starts, the deodorization and sterilization of the inside of the refrigerator is performed, and interior temperature is equalized at the same time, thus improving cooling efficiency.

On the other hand, in such cases as where the odor of the foods and the bacteria in the refrigerator are removed after the operation of deodorization and sterilization carried out for a given period of time, or where taking foods in and out of the refrigerator is not relatively frequent and thus there is no need for deodorization or sterilization, it is possible, by turning OFF the switch 254 to a stop side, to achieve energy saving through reduced power consumption and silent operation through stopping the fan sound.

Thus, by turning the switch 254 ON and OFF according to the situation of the user, it is possible to efficiently deodorize and sterilize the inside of the refrigerator.

### (Eighth Embodiment)

FIG. 33 is a control diagram according to the present invention.

As the figure shows, when a switch 254 is turned ON to an operation side, a surface of a support 201 is irradiated with light energy (blue light or an ultraviolet ray) emitted from the irradiation unit 202 onto the support 201, and silver oxide having an absorption spectrum in these wavelength ranges is excited by the blue light energy, so that OH radial is generated from moisture in the air.

On the other hand, since an air blowing unit 203 is controlled so as to repeatedly start and stop operation, the air which contains odors (such as an organic substance) and bacteria is introduced into a sterilization apparatus 200 only when the air blowing unit 203 is in operation.

When the introduced air passes licking the surface of the support 201, the bacteria are captured by the support 201 and then lysed by this OH radical.

Furthermore, when the air passes through an absorption filter 204, chemical change of the odor components is induced to perform deodorization. In addition, the air, which is now clean, deodorized and sanitized air, is blown toward the cooler 110 that is cooled and undergoes heat exchange, so that the clean cool air circulates in a refrigerating compartment 102.

Thus, since the air blowing unit 203 operates intermittently, it is possible to save power consumption.

### (Ninth Embodiment)

FIG. 34 is a control diagram according to the present invention.

As the figure shows, when a switch 254 is turned ON to an operation side, an air blowing unit 203 repeatedly starts and stops operation, and an irradiation unit 202 is controlled such that it operates only when the air blowing unit 203 is in operation.

When the air blowing unit 203 is in operation, a surface of a support 201 is irradiated with light energy (blue light or an ultraviolet ray), and silver oxide having an absorption spectrum in these wavelength ranges is excited by the blue light energy, so that OH radial is generated from moisture in the air.

At this time, the air containing odors (such as an organic substance) and bacteria is introduced into a sterilization apparatus 200. When the introduced air passes licking the surface of the support 201, the bacteria are captured by the support 201 and then lysed by this OH radical.

Furthermore, when the air passes through an absorption filter 204, chemical change of the odor components is induced to undergo deodorization.

Then, the air, which is now clean, deodorized and sanitized air, is blown toward a cooler 110 to cause heat exchange, so that the clean cool air circulates in a refrigerating compartment 102.

Thus, the air blowing unit 203 and the irradiation unit 202 repeatedly start and stop in collaboration, thus making it possible to save power consumption and extend the life of the irradiation unit 202.

The refrigerator includes a main body which is made of a heat-insulating material and in which a storage compartment is formed, a door attached to an opening provided on the main body so as to be openable and closable, a cooling unit which cools air inside the main body to generate cool air, an air circulation path forming unit provided in the storage compartment, a fan which forcibly circulates the air in the air circulation path forming unit, a support having a photocatalyst and an absorption filter, and an irradiation unit which irradiates the support with excitation light which excites the photocatalyst, and a switch for starting and stopping an operation of the fan and the irradiation unit is provided, and when the switch is turned ON to an operation side, odors from food and bacteria inside the refrigerator are collected into a flow path forming unit to be deodorized and sterilized by the support immediately before the air is blown into the storage compartment, thereby making it possible to blow clean air into the refrigerator and further to improve uneven internal temperature distribution which is a problem of the refrigerator.

Furthermore, when the switch is turned ON to the operation side, the fan is controlled so as to alternate start and stop repeatedly, thus making it possible to save power consumption through the starts and stops repeated by the fan.

Furthermore, by providing the irradiation unit which is controlled in collaboration with the operation of the fan so as to emit excitation light, the irradiation frequency of the excitation light is decreased, thus allowing extended life of the irradiation unit.

### (Tenth Embodiment)

FIG. 35 is a center longitudinal sectional view of a refrigerator in an embodiment of the present invention.

FIG. 36 is a center longitudinal sectional view of a sterilization and deodorization apparatus mounted to the refrigerator in the embodiment of the present invention.

FIG. 37 is an outer bottom view of the sterilization and deodorization apparatus mounted to the refrigerator in the embodiment of the present invention.

A refrigerator 100 shown in these figures includes: a storage compartment made up of a heat-insulating main body 101 and a heat-insulating door 107, and a refrigerating compartment 102 is provided in an upper part and a freezing compartment 103 is provided in a lower part. The refrigerating compartment 102 includes shelf plates 46 on which food is placed, and a cooler 110 dedicated for the refrigerating compartment is provided in a back wall at the back of the shelf plates. Likewise, cases 48 for storing food are provided in the freezing compartment 103, and the cooler 110 and a cooling fan 121 both dedicated for the freezing compartment 103 are provided in a cooling room 115 formed at the back of the cases 48. In addition, in a rear lower part of the heat-insulating main body 101, a compressor 114 of a refrigeration cycle is provided. In addition, a sterilization apparatus 200 is provided in the ceiling of the refrigerating compartment 102.

The sterilization apparatus 200 includes: a housing 250, an air blowing unit 203, an absorption filter 204, a support 201 which is blended with or on which a photocatalyst is supported, and an irradiation unit 202 which emits light for exciting the photocatalyst.

Inside the housing 250, a cool-air path is formed. The housing 250 includes an inlet 251 which opens forward and an outlet which opens rearward. The housing 250 is a case in which to house the whole sterilization apparatus 200, and part thereof also includes a function as a cover 206.

The air blowing unit 203 is referred to as a propeller-type axial-flow fan having a blade shape, and is provided adjoining the inlet 251 on a down-stream side and leaning forward.

The absorption filter 204 is a blend of manganese oxide and silicon- or sodium-oxide, and is formed in a honeycomb shape so as to have a large surface area. The absorption filter 204 includes a function to absorb and deodorize odor components.

The support 201 is formed with a non-woven fabric, which is made of porous resin fibers which allows more contact with cool air, and into which titanium oxide as a photocatalyst is kneaded.

The irradiation unit 202 includes an LED as a light source 207 and a light source cover 212 which transmits light. The light source 207 emits an excitation light having a wavelength 400 nm or below in the ultraviolet range.

The irradiation unit 202 and the support 201 are provided to face each other, sandwiching an air path downstream from the air blowing unit 203 in between.

The operation and effects of the refrigerator 100 thus configured will be described below.

First, the operation of cooling the refrigerating compartment 102 will be described.

The compressor 114, when in operation, cools the cooler 110 to a low temperature, so that the air in an upper back of the refrigerating compartment 102 is cooled. Fresh air thus generated has high specific gravity and thus goes down along the back wall of the refrigerating compartment 102 to be distributed onto each of the shelf plates 46, and cools the food placed thereon.

The cool air after cooling the food has low specific gravity and thus goes up along the inner wall of the insulating door 107 and returns to an uppermost part of the refrigerating compartment 102.

The cool air having thus returned is cooled again by the cooler 110, and convectively flows inside the refrigerating compartment 102 as fresh air again. As described above, the method for cooling the storage compartment by using natural convective air is referred to as the direct cooling method.

Next, the operation of cooling the freezing compartment 103 is described.

The compressor 114, when in operation, cools the cooler 110 to a low temperature, so that the air in the upper back of the refrigerating compartment 102 is cooled. Fresh air thus generated is blown into the freezing compartment 103 by the operation of the cooling fan 121 for the freezing compartment, and cools the food stored in the cases 48.

The cool air, after cooling the food, returns to the cooling room 115 to be cooled by the cooler 110 again, and is blown into the freezing compartment 103 again. Thus, the method for cooling the storage compartment by circulating the cool air with the cooling fan 121 for the freezing compartment is referred to as the indirect cooling method.

Next, the sterilization operation will be described. Inside the refrigerating compartment 102 where natural convective air is generated, when the air blowing unit 203 equipped inside the sterilization apparatus 200 is in operation, part of the cool air having retuned to the uppermost part of the refrigerating compartment 102 is sucked through the inlet 251. This sucked cool air passes through the air blowing unit 203 to be sent to a space between the support 201 and the irradiation unit 202, where bacteria and others floating in the air are supposed to be killed.

While the sterilization apparatus 200 is in operation as above, when, for example, opening the heat-insulating door 107 to look inside the storage compartment 102, the user can see the fan rotating at the back of the inlet 251 and the light of the irradiation unit 202 through gaps between the blades of the air blowing unit 203, so that the user can easily recognize that the sterilization apparatus 200 is in operation. Particularly, in the present embodiment, the refrigerating compartment 102 is provided in an upper part and the air blowing unit 203 is provided leaning forward. This structure provides high visibility as the user's line of sight comes right in front of the air blowing unit 203 simply by looking inside the refrigerating compartment 102 with quite a natural posture.

Meanwhile, a specific sterilization mechanism is shown below.

When an ultraviolet energy is emitted from the irradiation unit 202, movement (excitation) of electrons occurs inside the titanium oxide kneaded into the support 201. This movement of electrons generates a portion in which electrons are missing. This portion is referred to as an electron hole.

This electron hole moves from inside to the surface of the titanium oxide, and causes chemical reaction of the substance attached to the surface. Specifically, the electron hole takes away electrons from the OH minus contained the water molecule in the atmosphere to generate OH radical that is a type of active oxygen.

The generated OH radical, which has a significantly unstable and powerful oxidative power, tries to stabilize itself by taking away electrons from an organic compound which is around, such as bacteria.

The organic compound, whose electrons have thus been taken away, loses its binding energy to become decomposed. Due to the effect as described above, the organic compound such as bacteria is finally turned into a nontoxic substance such as water and carbon dioxide, and diverges into the atmosphere.

Next, the deodorization operation will be described. The cool air, which is sterilized by the effects of the support 201 and the irradiation unit 202, is sent to the absorption filter 204 which absorbs odor components. Most of the odor components that are present in the refrigerator are sulfur gasses composed of methyl mercaptan which is represented by a foul odor of rotten onions. When this methyl mercaptan contacts a manganese catalyst, dehydrogenation reaction is caused, and the methyl mercaptan turns into dimethyl disulfide which has small odor intensity. Accordingly, by forming the manganese catalyst into a honeycomb shape which allows sufficient contact with air, it is possible to perform deodorization effectively.

Thus, clean cool air, which has undergone sterilization and deodorization, is returned by the cooler 110 after blown out through the outlet 252, and naturally flows convectively in the refrigerating compartment 102. The cool air, while convectively flowing, mixes with germs and odors inside the refrigerating compartment 102 and is guided to the sterilization apparatus 200 again, to be affected by the sterilization and deodorization action and regenerated as clean cool air. In the mechanism, by repeating this circulation many times, the whole air inside the refrigerating compartment 102 is turned into a clean state with little bacteria and foul odors.

Accordingly, as is clear from the above description, it is possible to complete sterilization and deodorization within a shorter time when a larger amount of air is blown into the sterilization apparatus 200. In other words, higher-performance deodorization and sterilization can be achieved.

In the present embodiment, since the irradiation unit 202 and the support 201 are provided facing each other and sandwiching the cool-air path in between, the structure hardly increases resistance to the air path, thus making it possible to secure a sufficient amount of air passing thorough the sterilization apparatus 200.

With the operation as described above, the cool air in the refrigerating compartment 102 is deodorized and sterilized, so that a clean state with less foul odor and bacteria is maintained.

Meanwhile, in the refrigerator 100 in the present embodiment, the back wall surface of the refrigerating compartment 102 in which the cooler 110 is provided is consistently wet with dew condensation water. There is a possibility that this dew condensation water will cause a trouble such as breakdown or electric leakage when it attaches to an electric component. Thus, two electronic components likely to have troubles caused by an attachment of the dew condensation water are the air blowing unit 203 and the irradiation unit 202, and therefore it is important to provide these two with a splash-proof structure.

Regarding the splash-proof structure, in the case of the air blowing unit 203, first, splash-proof air blowing units such as the cooling fan 121 for the freezing compartment, for example, have already become widely popular and achieved significant market performance. Thus, it is only necessary to select a desired one from among such air blowing units.

On the other hand, in the case of the irradiation unit 202, as a measure for splash proofing, the light source cover 212 is provided circumferentially surrounding the light source 207 that is to be the light source. The light source 207 has an advantage of being compact and emitting a small amount of heat, and thus no abnormal increase of temperature affecting the life of components occurs even when continuously lighting the light source 207 in such an enclosed space as in the light source cover 212.

As described above, since the air blowing unit 203 and the irradiation unit 202 can be splash-proof by taking a relatively easy measure, it is possible to collectively provide structural components of the sterilization apparatus 200 including the air blowing unit 203 and the irradiation unit 202 near the back wall surface of the refrigerator 102, thus rendering the deodorization and sterilization apparatus more compact than ever.

Furthermore, the air blowing unit 203 and the irradiation unit 202 both operated with a direct-current low-voltage electric circuit are widely used, and thus the sterilization apparatus 200 in which these components are used is much safer without requiring a high-voltage circuit.

As described above, the present embodiment is characterized by: including a refrigerating compartment for storing fresh food, a cooler provided in the back wall of the refrigerating compartment, and a deodorization and sterilization apparatus provided on the ceiling of the refrigerating room; and being structured with a housing having an inlet that opens forward and an outlet that opens rearward, an air blower which sucks air from the refrigerating compartment through the inlet and discharges the air through the outlet, an absorption filter which absorbs odor components in the air, a support compounded with a photocatalyst, and a light-emitting object which emits light for exciting the photocatalyst. With these characteristics, two electric components, that is, the air blower and the light-emitting object, are under influence of dew condensation water, and since these two can be splash-proof by taking a relatively easy measure, it is possible to collectively provide, near the back wall surface of the refrigerating compartment, the constituent components of the deodorization and sterilization apparatus including the air blower and the light-emitting object. Thus, the deodorization and sterilization apparatus is rendered more compact than ever with less projection to the storage space, so that usability is improved.

Additionally, the air blower and the light-emitting object both operated with a direct-current low-voltage electric circuit are widely used. Thus, when these components are used, the deodorization and sterilization apparatus does not need a high-voltage electric circuit, thus improving safety because an ignition source for a flammable refrigerant is eliminated.

In addition, the present embodiment is characterized by the air blower being provided adjacent to the inlet. This characteristic allows the user to directly see and confirm that the blades are actually rotating, thus making it possible to remove anxieties about troubles and so on and make the user feel reassured.

In addition, the preset embodiment is characterized by providing the air blower leaning forward. This characteristic reduces a distance of the air blower in a height direction, thus achieving a compact structure of the sterilization apparatus as a whole and making the frontal part of the air blower closer to the user's line of sight in an opposite direction, so that the user can see, more easily, the blades of the air blower rotating.

In addition, the present embodiment is characterized by providing the light-emitting object and the support such that they face each other sandwiching an air path downstream from the air blower in between. This characteristic reduces resistance to the air path and allows obtainment of a sufficient amount of air passing through the deodorization and sterilization apparatus, thus making it possible to improve the deodorization and sterilization performances.

In addition, in the present embodiment, by placing the light-emitting object in an upper side of the air path, dew condensation water, which attaches to the cover and so on provided around the light-emitting object, drips down due to gravity. Thus, the light source is not blocked by the dew condensation water, and it is possible to constantly ensure a given amount of light, thus preventing degradation of the sterilization performance.

In addition, in the present embodiment, a virtual line which connects the center of the light source of the light-emitting object and the center of the air blower is extended toward the refrigerating compartment side so as to intersect with the door of the refrigerating compartment. This allows the user to visually recognize the status of irradiation by the light-emitting object which appears in the back of the air blower through the gaps between the blades, thus making it possible to constantly check the trouble due to a breakdown or end of life.

Note that in the present embodiment, it is assumed that the photocatalyst blended into the support is titanium oxide and the light source of the light-emitting object is an LED having an ultraviolet range. Instead of these, however, the photocatalyst may also be silver oxide and the light source may also be an LED having a blue color wavelength range approximately 450 nm to 470 nm. This slightly reduces the sterilization performance, but produces an advantageous effect of allowing use of an inexpensive and long-life LED.

### (Examples)

Hereinafter, Examples are described with reference to the drawings. Note that the present invention is not limited by this embodiment.

FIG. 38 shows a cross-sectional view of an air purifying apparatus which is part of the sterilization apparatus.

Here, the air purifying apparatus is an apparatus which includes a support 201 and an irradiation unit 202 and purifies air using the effect of the photocatalyst.

As the figure shows, the air purifying apparatus 250 includes a housing 250, the support 201, and the irradiation unit 202. In addition, in the housing 250, the support 201 and a plurality of light sources 207 as the irradiation unit 202 are attached. In addition, the housing 250 functions as a flow path forming unit 124, and a flow path 4 is formed inside the housing 250. The flow path 4 is a flow path through which the air from outside of the air purifying apparatus 258 can be sucked and discharged. The light sources 207 is a light-emitting diode (LED).

Used for the support 201 is polyester resin, on which 2 g/m² of silver zirconium phosphate compounded with 3-wt% silver is applied using acrylic binder. As the support for the silver, it is possible to use a support such as zeolite, silica gel, glass, calcium phosphate, silicate salt, and titanium oxide. In the case of using silver zirconium phosphate, silver is characteristically difficult to dissolve in water, and thus it is preferable to use silver zirconium phosphate when the support 201 is specified to be washed with water.

In the present embodiment, silver zirconium phosphate is applied as the support 201 by using a binder, but it is also possible to form the support 201 from polyester into which the silver zirconium phosphate is kneaded.

In addition, in the present embodiment, the support 201 is sheet-shaped but is not limited to this shape. It is also possible to form the support 201 into a honeycomb shape, a pleated shape, a non-woven fabric, and so on.

### (Experimental Example 1)

First, for a comparative example 1, a sheet-shaped support 201 which did not contain silver was prepared. An apparatus having the same shape as the air purifying apparatus 258 shown in FIG. 38 was used, and a silver-free dummy of the support 201 was sprayed with an emulsion of staphylococcus aureus, and then was light-shielded and left for three hours. After that, the dummy was taken out and washed out with normal saline so that the number of staphylococcus aureus in the washout liquid was approximately 1.0 X 10⁶ CFU/cc. The method for measuring the number of staphylococcus aureus was to perform plate-dilution on a normal agar culture medium and count the number of colonies on the agar culture medium after culturing the staphylococcus aureus for 48 hours at 35 °C.

Next, a sheet-shaped polyester resin support, into which 2-wt% silver-supported zirconium phosphate compounded with 3-wt% silver was kneaded, was sprayed with the same amount of emulsion of staphylococcus aureus as in the first comparative example 1, and then was light-shielded and left for three hours. After that, the support 201 was taken out and washed out with normal saline. The number of staphylococcus aureus in the washout liquid proved 1.0 × 10³ CFU/cc, and it was confirmed that the sheet produced antibacterial performance as compared to the comparative example 1.

Furthermore, the same experiment was performed using, as the light source 207, LEDs having different wavelengths. The irradiation time was set to three hours, and the light was shielded so that the light from outside would not come in. The average irradiation illuminance over the support 201 was adjusted to 150 LUX. (Table 1) shows a center peak wavelength of the LED used in the experiment and the number of cultured staphylococcus aureus which was washed out from the irradiated support 201.

**(Table 1)**

| Test No. | Center peak wavelength (nm) | Cultured staphylococcus aureus count (CFU/cc) |
|---|---|---|
| Test 1 | 360 | 0.6 × 10 |
| Test 2 | 380 | 1.0 × 10 |
| Test 3 | 400 | 2.0 × 10 |
| Test 4 | 450 | 4.0 × 10 |
| Test 5 | 500 | 7.0 × 10 |
| Test 6 | 520 | 9.0 × 10 |
| Test 7 | 530 | 1.0 × 10³ |
| Test 8 | 540 | 1.0 × 10³ |

As is clear from the results in (Table 1), in the case of tests 1 to 6 where the support was irradiated with light having a central peak wavelength equal to or below 520 nm, the number of cultured staphylococcus aureus significantly decreased as compared to tests 7 and 8 having a center peak wavelength above 520 nm. Here, it was proved that whereas the sterilization effect was insufficient due to lack of energy in the case of irradiation with the light having a center peak wavelength equal to or above 530 nm, sufficient light energy was obtained in the case of irradiation with the light having a center peak wavelength equal to or below 520 nm, thus increasing the antibacterial activity of the silver and producing a more significant sterilization effect.

In addition, in tests 1 to 4, in the case of continuous operation for 2000 hours by using the support 201 of which the surface was applied with a mixture of acrylic binder and silver-supported zirconium phosphate, the support 201 deteriorated in tests 1 and 2 because the resin molecular structure was destroyed by ultraviolet rays, causing the film applied on the surface of the support 201 to peal off. In tests 3 and 4, since the light did not have a wavelength of an ultraviolet range, no resin deterioration was caused, and the film was not caused to peal off.

### (Experimental Example 2)

Next, in the same air purifying apparatus as in the tests 1 and 3, the illuminance over the support 201 was changed by adjusting a distance between the light source 207 and the support 201. As in Experimental Example 1, the change of the bacteria applied to the filter material was studied. (Table 2) shows the illuminance over the support 201 and the number of cultured bacteria washed out from the support 201. The test condition was set to the same as in Experimental Example 1.

**(Table 2)**

| Test No. | Illuminance (Lux) | Cultured staphylococcus aureus count (CFU/cc) |
|---|---|---|
| Test 11 | 80 | 9.8 × 10² |
| Test 12 | 90 | 8.0 × 10² |
| Test 13 | 100 | 5.0 × 10 |
| Test 14 | 110 | 3.0 × 10 |
| Test 15 | 120 | 2.9 × 10 |
| Test 16 | 130 | 2.5 × 10 |
| Test 17 | 140 | 2.2 × 10 |
| Test 18 | 150 | 2.0 × 10 |

As is clear from (Table 2), when the illuminance in tests 13 to 18 was equal to or above 100 Lux, the number of cultured bacteria was far smaller than in tests 11 and 12 when the illuminance was equal to or below 90 Lux. In contrast, it can be considered that a sufficient sterilization effect was not produced due to lack of sufficient light energy when the illuminance was equal to or below 90 Lux. This proves that sufficient light energy was obtained through the irradiation with the light having illuminance equal to or above 100 Lux, so that the antibacterial activity of the silver was increased to produce a more significant sterilization effect.

FIG. 39 is a cross-sectional view showing indoor equipment of an air conditioner.

As the figure shows, the air conditioner 15 includes: a housing 250, a cooler 110, a cooling fan 121, a wind direction louver 13, and the air purifying apparatus 258 used in the first embodiment.

First, for a comparative example 2, a sheet-shaped dummy of the support 201 which did not contain silver was prepared. In the air purifying apparatus 258, the silver-free dummy of the support 201 was sprayed with an emulsion of staphylococcus aureus, and then was light-shielded and left for three hours. After that, the dummy was taken out and washed out with normal saline such that the number of staphylococcus aureus in the washout liquid was approximately 1.0 X 10⁶ CFU/cc. The method for measuring the number of staphylococcus aureus was to perform plate-dilution on a normal agar culture medium and count the number of colonies on the agar culture medium after culturing the staphylococcus aureus for 48 hours at 35 °C. Note that the test was performed without operating the air conditioner 15.

### (Experimental Example 3)

In Experimental Example 3, the support 201, which was a sheet-shaped polyester resin filter on which 2 g/m² of silver zirconium phosphate compounded with 3-wt% silver was applied using acrylic binder, was sprayed with the same amount of emulsion of staphylococcus aureus as in the comparative example 2, and then was light-shielded and left for three hours. After that, the support 201 was taken out and washed out with normal saline. The number of staphylococcus aureus in the washout liquid proved 1.0 × 10³ CFU/cc, and it was confirmed that the sheet produced antibacterial performance. Note that the test was performed without operating the air conditioner 15.

### (Experimental Example 4)

Next, in Experimental Example 4, a test was further conducted using an LED having a center peak wavelength 450 nm as the light source 207. More specifically, in the air purifying apparatus 258, the support 201, which was a sheet-shaped polyester resin filter on which 2 g/m² of silver zirconium phosphate compounded with 3-wt% silver was applied using acrylic binder, was sprayed with the same amount of emulsion of staphylococcus aureus as in the comparative example 2, and then was light-shielded and left for three hours. After that, the support 201 was taken out and washed out with normal saline. Note that during the test, the light was shielded so that the light from outside would not come in. In addition, the average illuminance over the support 201 was adjusted to 150 LUX. The number of staphylococcus aureus washed out from the irradiated support 201 proved 4.0 × 10, and it was confirmed that the sheet produced a sterilization effect.

Next, after performing cooling operation for one hour with the air conditioner 15 used in Experimental Example 4 and stopping the operation, a test was conducted using, as the light source 207, an LED having a center peak wavelength 450 nm. The irradiation time was set to three hours, and the light was shielded so that the light from outside would not come in. The average irradiation illuminance over the support 201 was adjusted to 150 LUX. The number of cultured staphylococcus aureus washed out from the support 201 after the three-hour irradiation proved 1.0 × 10.

It can be considered that at this point, the humidity inside the air conditioner 15 increased after the cooling operation, and that the activity is increased by the presence of ample moisture necessary for the photocatalytic action of silver at the time of irradiation.

In addition, after performing warming operation for one hour with the air conditioner 15 used in Experimental Example 4 and stopping the operation, a test was conducted using, as the light source 207, an LED having a center peak wavelength 450 nm. The irradiation time was set to three hours, and the light was shielded so that the light from outside would not come in. The average irradiation illuminance over the support 201 was adjusted to 150 LUX. The number of cultured staphylococcus aureus washed out from the support 201 after the three-hour irradiation became 1.2 × 10.

Here, it can be considered that the temperature inside the air conditioner 15 became high, so that the photocatalytic action of the silver was increased when irradiated with light.

FIG. 40 shows a cross-sectional view of the air purifying apparatus in an embodiment of the present invention.

In the figure, a supply unit 20 is attached inside the housing 250. The supply unit 20 is a supply unit which supplies water into the housing 250. Used for the support 201 was polyester resin, on which 2 g/m² of silver zirconium phosphate compounded with 3-wt% silver was applied using an acrylic binder. The air purifying apparatus shown in the figure was provided in the refrigerating compartment of the refrigerator.

First, for a comparative example 3, a sheet-shaped dummy of the support 201 which did not contain silver was prepared. In the air purifying apparatus shown in the figure, the silver-free dummy of the support 201 was sprayed with an emulsion of staphylococcus aureus, and then was light-shielded and left for three hours. After that, the dummy was taken out and washed out with normal saline such that the number of staphylococcus aureus in the washout liquid was approximately 1.0 X 10⁶ CFU/cc. The method for measuring the number of staphylococcus aureus was to perform plate-dilution on a normal agar culture medium and count the number of colonies on the agar culture medium after culturing the staphylococcus aureus for 48 hours at 35 °C. Note that the test is conducted without operating the refrigerator.

### (Experimental Example 5)

In Experimental Example 5, a sheet-shaped polyester resin support on which 2 g/m² of silver zirconium phosphate compounded with 3-wt% silver was applied using acrylic binder, was sprayed with the same amount of emulsion of staphylococcus aureus as in the comparative example 2, and then was light-shielded and left for three hours. After that, the support 201 was taken out and washed out with normal saline. The number of staphylococcus aureus in the washout liquid proved 1.0 × 10³ CFU/cc, and it was confirmed that antibacterial performance was produced. Note that the test was conducted without operating the refrigerator.

### (Experimental Example 6)

Next, in Experimental Example 6, a test was further conducted using an LED having a center peak wavelength 470 nm as the light source 207. In other words, in the air purifying apparatus 258, the support 201, which was a sheet-shaped polyester resin filter on which 2 g/m² of silver zirconium phosphate compounded with 3-wt% silver was applied using acrylic binder, was sprayed with the same amount of emulsion of staphylococcus aureus as in the comparative example 3, and then was light-shielded and left for three hours. After that, the support 201 was taken out and washed out with normal saline. Note that during the test, the light was shielded so that the light from outside would not come in. In addition, the average irradiation illuminance over the support 201 was adjusted to 150 LUX. The number of staphylococcus aureus washed out from the irradiated support 201 proved 3.9 × 10. It was confirmed that, compared to Experimental Example 5, the antibacterial performance was increased by the irradiation with the light.

### (Experimental Example 7)

Next, in Experimental Example 7, after performing refrigerating operation for one hour with the refrigerator and stopping the operation, a test was conducted using, as the light source 207, an LED having a center peak wavelength 470 nm. Note that atomized ice-making water was already supplied from the supply unit 20. The irradiation time was set to three hours, and the light was shielded so that the light from outside would not come in. The average illuminance over the support 201 was adjusted to 150 LUX. The number of staphylococcus aureus washed out from the filter after three-hour irradiation proved 0.9 × 10, and it was confirmed that, compared to Experimental Example 6, the antibacterial performance was further increased.

In Experimental Example 7, it can be considered that the humidity inside the air purifying apparatus increased, and that the activity was increased by the presence of ample moisture necessary for the photocatalytic action of silver at the time of irradiation.

### Industrial Applicability

As described thus far, according to the present invention, a deodorization and sterilization support and absorption filter is provided in an air circulation and sterilization apparatus, with which the air is cleaned immediately before blown into the storage compartment. Thus, the present invention is applicable to refrigerating equipment in general such as a refrigerator, an air conditioner, and an air purifier.

## Claims

1. A refrigerator including a heat-insulating main body which is made of a heat-insulating material and in which a storage compartment is formed, a door attached to an opening provided on said heat-insulating main body so as to be openable and closable, and a cooling unit configured to cool air inside said heat-insulating main body, said refrigerator comprising:
a support which is placed in said storage compartment and on which a photocatalyst is supported;
an irradiation unit configured to irradiate said support with excitation light which excites the photocatalyst; and
an air blowing unit configured to forcibly blow the cool air inside said storage compartment toward said support.

2. The refrigerator according to Claim 1, comprising
a flow path forming unit configured to form a flow path for the cool air blown by said air blowing unit,
wherein said support is provided in the flow path formed by said flow path forming unit.

3. The refrigerator according to Claim 2, comprising
an absorption filter which absorbs and holds an odor that is present inside said storage compartment,
wherein said absorption filter is placed downstream from said support in the flow path.

4. The refrigerator according to Claim 1,
wherein the photocatalyst supported on said the support contains silver.

5. The refrigerator according to Claim 4,
wherein the photocatalyst supported on said support is silver oxide.

6. The refrigerator according to Claim 4,
wherein the photocatalyst supported on said support is silver phosphate zirconium.

7. The refrigerator according to Claim 1,
wherein said irradiation unit includes a light source which emits light having a wavelength of an ultraviolet range or a blue wavelength range.

8. The refrigerator according to Claim 7,
wherein said light source emits light having a wavelength determined within a range of above 400 nm to 520 nm.

9. The refrigerator according to Claim 7,
wherein said light source is provided such that at least part of a surface of said support has a luminance of 100 Lux or above.

10. The refrigerator according to Claim 1,
wherein said support includes an absorption unit which absorbs and holds an odor in said storage compartment.

11. The refrigerator according to Claim 1, further comprising
a cover attached to an inner wall of said heat-insulating main body and covering said support, said irradiation unit, and said air blowing unit.

12. The refrigerator according to Claim 11, comprising
a heat-insulating material which is provided between said cover and one of said support, said irradiation unit, and said air blowing unit.

13. The refrigerator according to Claim 11,
wherein said heat-insulating main body includes a concave portion which opens toward said storage compartment, and
one of said support, said irradiation unit, and said air blowing unit is attached to said concave portion.

14. The refrigerator according to Claim 11, further comprising
a lighting unit which is covered with said cover and configured to illuminate an inside of said heat-insulating main body.

15. The refrigerator according to Claim 14,
wherein said lighting unit is attached closer to a door side than said support, said irradiation unit, and said air blowing unit.

16. The refrigerator according to Claim 15,
wherein said lighting unit has directivity toward an irradiation direction of light and is attached to said heat-insulating main body such that the light is directed from the door side to an innermost side of said heat-insulating main body.

17. The refrigerator according to Claim 15,
wherein said heat-insulating main body includes a concave portion which opens toward said storage compartment, and
said lighting unit is attached to said concave portion.

18. The refrigerator according to Claim 11, further comprising
a second cover which covers said irradiation unit.

19. The refrigerator according to Claim 11,
wherein said support is placed between said irradiation unit and said cover, and
said cover includes:
a support member provided in said cover in a protruded state and supporting said support; and
a projection which holds said cover and said support with a predetermined space.

20. The refrigerator according to Claim 11, further comprising
a third cover placed at a predetermined distance from said cover.

21. The refrigerator according to Claim 11,
wherein said cover includes a through hole which connects an inside of said storage compartment and an inside of said sterilization apparatus.

22. The refrigerator according to Claim 1, further comprising
a flow path forming unit configured to form a flow path for the cool air blown by said air blowing unit, wherein said flow path forming unit has, in the flow path for the cool air, a narrow portion of which a cross-sectional area is narrower than a cross-sectional area of another portion, and
said support is placed in said narrow portion.

23. The refrigerator according to Claim 22,
wherein said air blowing unit has an axial-flow fan, which is placed such that a rotational axis of said axial-flow fan intersects with a direction in which the cool air passes through the flow path.

24. The refrigerator according to Claim 23, comprising
an absorption filter, which absorbs and holds an odor in said storage compartment,
wherein said absorption filter is placed downstream from said support in the flow path such that a direction in which the cool air passes through said absorption filter intersects with the direction in which the cool air passes through the flow path.

25. The refrigerator according to Claim 1,
wherein said air blowing unit, said support, and said cooling unit are placed in order, starting from upstream of a flow of the cool air generated by said air blowing unit.

26. The refrigerator according to Claim 25, comprising
an absorption filter which absorbs an odor in said storage compartment,
wherein said absorption filter is placed downstream from said support and upstream from said cooling unit.

27. The refrigerator according to Claim 25,
wherein said cooling unit is attached to an inner back wall of said heat-insulating main body, and
said support, said irradiation unit, and said air blowing unit are attached on a ceiling of said heat-insulating main body.

28. The refrigerator according to Claim 1,
wherein a switch for starting and stopping operation of said air blowing unit and said irradiation unit is provided.

29. The refrigerator according to Claim 28,
wherein when said switch is turned ON to an operation side, said air blowing unit is controlled so as to alternate start and stop repeatedly.

30. The refrigerator according to Claim 1, further comprising
a housing having an inlet which opens forward and an outlet which opens rearward, said housing accommodating said air blowing unit, said support, and said irradiation unit.

31. The refrigerator according to Claim 30,
wherein said air blowing unit is provided adjacent to said inlet.

32. The refrigerator according to Claim 30,
wherein said air blowing unit is provided tilting forward.

33. The refrigerator according to Claim 30,
wherein said irradiation unit and said support are provided facing each other and sandwiching an air path in between, the air path being located downstream from said air blowing unit.

34. The refrigerator according to Claim 30,
wherein said irradiation unit is placed on an upper side of the air path.

35. The refrigerator according to Claim 30,
wherein an extension of a virtual line intersects with said door of said storage compartment, the virtual line connecting a center of said light source of said irradiation unit and a center of said air blowing unit, and the extension being extended toward said storage compartment.

36. The refrigerator according to Claim 1, further comprising
a flow path forming unit into which the cool air is forcibly inserted,
wherein said support is placed in an outlet portion of said flow path forming unit.

37. The refrigerator according to Claim 36,
wherein said irradiation unit is placed opposite to said support at a predetermined distance.

38. The refrigerator according to Claim 36,
wherein the outlet portion of said flow path forming unit has an elbow portion inside which said support is placed.

39. The refrigerator according to Claim 38,
wherein said support is placed on an inner wall of said flow path forming unit, said inner wall being hit by the cool air from said elbow potion.

40. The refrigerator according to Claim 36,
wherein part of said flow path forming unit is structured with a light-transmissive member,
said irradiation unit is provided near said light-transmissive member at the back of said storage compartment, and
said irradiation unit doubles as an illumination for illuminating the inside of said refrigerator and an excitation light source for exciting the photocatalyst supported on said support.

41. The refrigerator according to Claim 36,
wherein said support is plate-shaped and placed such that a normal line to a face having a larger area of said support intersects with a flow direction of the cool air.

42. The refrigerator according to Claim 36,
wherein the support has an inflected shape and is placed in such a manner as to divert the flow direction of the cool air.

43. The refrigerator according to Claim 36,
wherein said support is plate-shaped and placed with a face having a larger area as a vertical face.

44. A sterilization apparatus including a heat-insulating main body which is made of a heat-insulating material and in which a storage compartment is formed, a door attached to an opening provided on said heat-insulating main body so as to be openable and closable, and a cooling unit configured to cool air inside said heat-insulating main body, said sterilization apparatus comprising:
a support on which a photocatalyst is supported;
an irradiation unit configured to irradiate said support with excitation light which excites the photocatalyst;
an air blowing unit configured to forcibly blow cool air inside said storage compartment toward said support; and
a flow path forming unit configured to form a flow path for the cool air blown by said air blowing unit,
wherein said support, said irradiation unit, and said air blowing unit are internally attached to said flow path forming unit.

45. The sterilization apparatus according to Claim 44, further comprising
a cover which covers said support, said irradiation unit, said air blowing unit, and said flow path forming unit.

46. The sterilization apparatus according to Claim 45, further comprising
a lighting unit configured to illuminate an inside of said heat-insulating main body.

47. The sterilization apparatus according to Claim 45, further comprising
a second cover which covers said irradiation unit.

48. The sterilization apparatus according to Claim 45,
wherein said support is placed between said irradiation unit and said cover, and
said cover includes:
a support member provided in said cover in a protruded state and supporting said support; and
a projection which holds said cover and said support with a predetermined space.

49. The sterilization apparatus according to Claim 45, further comprising
a third cover placed at a predetermined distance from said cover.

50. The sterilization apparatus according to Claim 45,
wherein said cover includes a through hole which connects an inside of said storage compartment and an inside of said sterilization apparatus.

51. The sterilization apparatus according to Claim 44,
wherein said flow path forming unit has, in the flow path for the cool air, a narrow portion of which a cross-sectional area is narrower than a cross-sectional area of another portion, and
said support is placed in said narrow portion, and said support, said irradiation unit, and said air blowing unit are attached to said flow path forming unit.
